# EUROPEAN PATENT APPLICATION

(11) **EP 2 738 263 A1**
(43) Date of publication of application: **04.06.2014**
(21) Application number: 12382477.3
(22) Date of filing: 30.11.2012
(51) Int. Cl.: C12Q 1/68

(54) **Methods for DNA rearrengements analysis**

(71) Applicant: Fundacion para la Investigacion del Hospital Clinico de la Comunidad Valenciana, 46010 Valencia (ES)
(72) Inventor: Chaves Martinez, Felipe J., E-46118 Serra, Valencia (ES); Garcia Garcia, M Ana Bárbara, E-46021 Valencia (ES); Blesa Lujan, Sebastián, E-46016 Tavernes Blanques- Valencia (ES)
(74) Representative: ABG Patentes, S.L.

(57) **Abstract**

The present invention is related to PCR-based methods for detection of gene rearrangements, such as cytogenetic abnormalities, chromosome rearrangements and copy number variations (CNV) polymorphisms, in a nucleic acid sequence, that comprise modified PCR primers and PCR products size and/or quantity analysis. The invention is also related to kits for nucleic acids rearrangement detection and to the use of said kits in applications such as diagnosis.

SEQUENCE LISTING

<110>  FUNDACIÓN PARA LA INVESTIGACIÓN DEL HOSPITAL CLÍNICO DE LA
       COMUNIDAD VALENCIANA

<120>  METHODS FOR DNA REARRENGEMENTS ANALYSIS

<130>  P8104EP00

<160>  84

<170>  PatentIn version 3.5

<210>  1
<211>  41
<212>  DNA
<213>  Artificial Sequence

<220>
<223>  QxRLDL-E15-5unv

<400>  1
aggtcaggat caacgctcaa gaggcacgtg gcactcagaa g                          41


<210>  2
<211>  43
<212>  DNA
<213>  Artificial Sequence

<220>
<223>  QxRLDL-E15-3unv

<400>  2
catcttgcat gatccaacct tcgacccgtc tctgggtgaa gag                        43


<210>  3
<211>  42
<212>  DNA
<213>  Artificial Sequence

<220>
<223>  QxRLDL-E4-5unv

<400>  3
aggtcaggat caacgctcaa gcagcttcca gtgcaacagc tc                         42


<210>  4
<211>  46
<212>  DNA
<213>  Artificial Sequence

<220>
<223>  QxRLDL-E4-3unv

<400>  4
catcttgcat gatccaacct tccagttttc ctcgtcagat ttgtcc                     46


<210>  5
<211>  42
<212>  DNA
<213>  Artificial Sequence

<220>
<223>  QxRLDL-E5-5unv

<400>  5
aggtcaggat caacgctcaa gtgtcctgtt ttccagctgt gg                    42


<210>  6
<211>  44
<212>  DNA
<213>  Artificial Sequence

<220>
<223>  QxRLDL-E5-3unv

<400>  6
catcttgcat gatccaacct tcaaatcatt tgcaagcagc aagg                  44


<210>  7
<211>  42
<212>  DNA
<213>  Artificial Sequence

<220>
<223>  Qx-p53_E-5unv

<400>  7
aggtcaggat caacgctcaa gaagcagtca cagcacatga cg                    42


<210>  8
<211>  47
<212>  DNA
<213>  Artificial Sequence

<220>
<223>  Qx-p53_E-3unv

<400>  8
catcttgcat gatccaacct tcaatttcct tccactcgga taagatg               47


<210>  9
<211>  41
<212>  DNA
<213>  Artificial Sequence

<220>
<223>  QxRLDL-E7-5unv

<400>  9
aggtcaggat caacgctcaa ggttcccacg tctgcaatga c                     41


<210>  10
<211>  46
<212>  DNA
<213>  Artificial Sequence

<220>
<223>  QxRLDL-E7-3unv

<400>  10

catcttgcat gatccaacct tcacttaaca gatggggaaa ctgagg                    46


<210>  11
<211>  46
<212>  DNA
<213>  Artificial Sequence

<220>
<223>  QxRLDL-E13-5unv

<400>  11
aggtcaggat caacgctcaa ggtgtttaac gggatttgtc atcttc                    46


<210>  12
<211>  47
<212>  DNA
<213>  Artificial Sequence

<220>
<223>  QxRLDL-E13-3unv

<400>  12
catcttgcat gatccaacct tctatcctct ggggacagta ggttttc                   47


<210>  13
<211>  46
<212>  DNA
<213>  Artificial Sequence

<220>
<223>  QxRLDL-E18-5unv

<400>  13
aggtcaggat caacgctcaa ggatagtttc cgctgtttac catttg                    46


<210>  14
<211>  43
<212>  DNA
<213>  Artificial Sequence

<220>
<223>  QxRLDL-E18-5unv

<400>  14
catcttgcat gatccaacct tcaggtctca ggaagggttc tgg                       43


<210>  15
<211>  45
<212>  DNA
<213>  Artificial Sequence

<220>
<223>  QxRLDL-PrC-5uv

<400>  15
aggtcaggat caacgctcaa gagtttgcaa aatcccagag aaatc                     45


<210>  16
<211>  45

<212>  DNA
<213>  Artificial Sequence

<220>
<223>  QxRLDL-PrC-3uv

<400>  16
catcttgcat gatccaacct tcaaaggcac atgtgagaaa cttgc                    45


<210>  17
<211>  51
<212>  DNA
<213>  Artificial Sequence

<220>
<223>  QxRLDL-PrA-5uv

<400>  17
aggtcaggat caacgctcaa gcattcgtga aatctaacta ggagtagtct g             51


<210>  18
<211>  49
<212>  DNA
<213>  Artificial Sequence

<220>
<223>  QxRLDL-PrA-3uv

<400>  18
catcttgcat gatccaacct tctgaattgt atctgtgtga aacacattg                49


<210>  19
<211>  42
<212>  DNA
<213>  Artificial Sequence

<220>
<223>  QxRLDL-E3-5n

<400>  19
aggtcaggat caacgctcaa gcctcagtgg gtctttcctt tg                       42


<210>  20
<211>  44
<212>  DNA
<213>  Artificial Sequence

<220>
<223>  QxRLDL-E3-3n

<400>  20
catcttgcat gatccaacct tcgaccccgt agagacaaag tcag                     44


<210>  21
<211>  46
<212>  DNA
<213>  Artificial Sequence

<220>
<223>  QxRLDL-E2-5

<400>  21
aggtcaggat caacgctcaa gaaatagaca caggaaacgt ggtcag                    46


<210>  22
<211>  47
<212>  DNA
<213>  Artificial Sequence

<220>
<223>  QxRLDL-E2-3

<400>  22
catcttgcat gatccaacct tcaaataaat gcatatcatg cccaaag                   47


<210>  23
<211>  42
<212>  DNA
<213>  Artificial Sequence

<220>
<223>  qxRLDL-E9-5

<400>  23
aggtcaggat caacgctcaa ggtcaggaag atgacgctgg ac                        42


<210>  24
<211>  43
<212>  DNA
<213>  Artificial Sequence

<220>
<223>  qxRLDL-E9-3

<400>  24
catcttgcat gatccaacct tcaggaggag agaagggcat cag                       43


<210>  25
<211>  43
<212>  DNA
<213>  Artificial Sequence

<220>
<223>  QxRLDL-E11-5

<400>  25
aggtcaggat caacgctcaa gtttcttcca gaattcgttg cac                       43


<210>  26
<211>  43
<212>  DNA
<213>  Artificial Sequence

<220>
<223>  QxRLDL-E11-3

<400>  26
catcttgcat gatccaacct tcattgggcc actgaatgtt ttc                       43

<210>  27
<211>  42
<212>  DNA
<213>  Artificial Sequence

<220>
<223>  Qx-p53_D-5

<400>  27
aggtcaggat caacgctcaa ggctcaagac tggcgctaaa ag                          42


<210>  28
<211>  47
<212>  DNA
<213>  Artificial Sequence

<220>
<223>  Qx-p53_D-3

<400>  28
catcttgcat gatccaacct tcagaggact catcaagttc agtcagg                     47


<210>  29
<211>  44
<212>  DNA
<213>  Artificial Sequence

<220>
<223>  QxRLDL-E8-5

<400>  29
aggtcaggat caacgctcaa gctacaagtg ccagtgtgag gaag                        44


<210>  30
<211>  47
<212>  DNA
<213>  Artificial Sequence

<220>
<223>  QxRLDL-E8-3

<400>  30
catcttgcat gatccaacct tctatgagtc tgtgcaaagt tcagagg                     47


<210>  31
<211>  41
<212>  DNA
<213>  Artificial Sequence

<220>
<223>  QxRLDL-E17-5

<400>  31
aggtcaggat caacgctcaa ggagctgggt ctctggtctc g                           41


<210>  32
<211>  47
<212>  DNA
<213>  Artificial Sequence

<220>
<223>  QxRLDL-E17-3

<400>  32
catcttgcat gatccaacct tcgtcaaagt tgatgctgtt gatgttc                    47


<210>  33
<211>  51
<212>  DNA
<213>  Artificial Sequence

<220>
<223>  QxRLDL-I1B-5uv

<400>  33
aggtcaggat caacgctcaa gaataatttg ttattgcttt tattgcctta g               51


<210>  34
<211>  52
<212>  DNA
<213>  Artificial Sequence

<220>
<223>  QxRLDL-I1B-3uv

<400>  34
catcttgcat gatccaacct tcaatacctt tatatgaaag attctggttc tg              52


<210>  35
<211>  46
<212>  DNA
<213>  Artificial Sequence

<220>
<223>  QxRLDL-E1n-5uv

<400>  35
aggtcaggat caacgctcaa ggaggtgaag acatttgaaa atcacc                     46


<210>  36
<211>  48
<212>  DNA
<213>  Artificial Sequence

<220>
<223>  QxRLDL-E1n-3uv

<400>  36
catcttgcat gatccaacct tcgtcattta cagcatttca atgtgagg                   48


<210>  37
<211>  42
<212>  DNA
<213>  Artificial Sequence

<220>
<223>  P53-215-5

<400>  37
aggtcaggat caacgctcaa gcttcttgca ttctgggaca gc                         42

<210>  38
<211>  46
<212>  DNA
<213>  Artificial Sequence

<220>
<223>  P53-215-3

<400>  38
catcttgcat gatccaacct tcattcaact ttgggacagg agtcag                    46


<210>  39
<211>  46
<212>  DNA
<213>  Artificial Sequence

<220>
<223>  P53-450-5

<400>  39
aggtcaggat caacgctcaa gaggtaggac ctgatttcct tactgc                    46


<210>  40
<211>  48
<212>  DNA
<213>  Artificial Sequence

<220>
<223>  P53-450-3

<400>  40
catcttgcat gatccaacct tccaggtaaa acagtcaaga agaaaacg                  48


<210>  41
<211>  46
<212>  DNA
<213>  Artificial Sequence

<220>
<223>  PR5

<400>  41
aggtcaggat caacgctcaa gagaagattg gctcttacca cttgtc                    46


<210>  42
<211>  47
<212>  DNA
<213>  Artificial Sequence

<220>
<223>  PR-3

<400>  42
catcttgcat gatccaacct tccgtatttt gaaagcagaa actaggc                   47


<210>  43
<211>  45
<212>  DNA

<213>  Artificial Sequence

<220>
<223>  E2-5

<400>  43
aggtcaggat caacgctcaa gcgcgttgaa gaagtacaaa atgtc                    45


<210>  44
<211>  50
<212>  DNA
<213>  Artificial Sequence

<220>
<223>  E2-3

<400>  44
catcttgcat gatccaacct tctcttccct agtatgtaag gtcaattctg               50


<210>  45
<211>  45
<212>  DNA
<213>  Artificial Sequence

<220>
<223>  E3-5

<400>  45
aggtcaggat caacgctcaa gcagttcctg acacagcaga cattt                    45


<210>  46
<211>  47
<212>  DNA
<213>  Artificial Sequence

<220>
<223>  E3-3

<400>  46
catcttgcat gatccaacct tctctgagaa agaatgaaat ggagttg                  47


<210>  47
<211>  49
<212>  DNA
<213>  Artificial Sequence

<220>
<223>  E4-5

<400>  47
aggtcaggat caacgctcaa gtgcatgtaa gttcagtttt catagatca                49


<210>  48
<211>  43
<212>  DNA
<213>  Artificial Sequence

<220>
<223>  E4-3

<400>  48
catcttgcat gatccaacct tcctgtgaag gccctttctt ctg                          43


<210>  49
<211>  46
<212>  DNA
<213>  Artificial Sequence

<220>
<223>  E5-5

<400>  49
aggtcaggat caacgctcaa ggaggttttc tactgttgct gcatct                       46


<210>  50
<211>  46
<212>  DNA
<213>  Artificial Sequence

<220>
<223>  E5-3

<400>  50
catcttgcat gatccaacct tctaaggtgt gagaccagtg ggagta                       46


<210>  51
<211>  45
<212>  DNA
<213>  Artificial Sequence

<220>
<223>  E7-5

<400>  51
aggtcaggat caacgctcaa gcaatttatt ttgtccatgg tgtca                        45


<210>  52
<211>  49
<212>  DNA
<213>  Artificial Sequence

<220>
<223>  E7-3

<400>  52
catcttgcat gatccaacct tcaccacaaa tacaaattat gaccaagat                    49


<210>  53
<211>  47
<212>  DNA
<213>  Artificial Sequence

<220>
<223>  E8-5

<400>  53
aggtcaggat caacgctcaa gaaagagaac ctttgtctat gaagctg                      47


<210>  54

<211>  43
<212>  DNA
<213>  Artificial Sequence

<220>
<223>  E8-3

<400>  54
catcttgcat gatccaacct tctacaccaa atcccaagtc gtg                         43


<210>  55
<211>  47
<212>  DNA
<213>  Artificial Sequence

<220>
<223>  E9-5

<400>  55
aggtcaggat caacgctcaa gcctatagtg tgggagatca agaattg                     47


<210>  56
<211>  47
<212>  DNA
<213>  Artificial Sequence

<220>
<223>  E9-3

<400>  56
catcttgcat gatccaacct tctctctttt cagtgcctgt taagttg                     47


<210>  57
<211>  42
<212>  DNA
<213>  Artificial Sequence

<220>
<223>  E10-5

<400>  57
aggtcaggat caacgctcaa ggttcagagg caacgaaact gg                          42


<210>  58
<211>  49
<212>  DNA
<213>  Artificial Sequence

<220>
<223>  E10-3

<400>  58
catcttgcat gatccaacct tctccaggaa gactttgttt atagacctc                   49


<210>  59
<211>  43
<212>  DNA
<213>  Artificial Sequence

<220>

<223>  E10B-5

<400>  59
aggtcaggat caacgctcaa gctaaccaaa cggagcagaa tgg                43


<210>  60
<211>  50
<212>  DNA
<213>  Artificial Sequence

<220>
<223>  E10B-3

<400>  60
catcttgcat gatccaacct tctttgaaca cttagtaaaa gaaccaggtg          50


<210>  61
<211>  46
<212>  DNA
<213>  Artificial Sequence

<220>
<223>  E11-5

<400>  61
aggtcaggat caacgctcaa gggtgtcctt tgcattcagt agtatg              46


<210>  62
<211>  47
<212>  DNA
<213>  Artificial Sequence

<220>
<223>  E11-3

<400>  62
catcttgcat gatccaacct tctgggtttc gaaggtttag cttattc             47


<210>  63
<211>  48
<212>  DNA
<213>  Artificial Sequence

<220>
<223>  E12-5

<400>  63
aggtcaggat caacgctcaa gttcatttaa tggaaagctt ctcaaagt            48


<210>  64
<211>  48
<212>  DNA
<213>  Artificial Sequence

<220>
<223>  E12-3

<400>  64
catcttgcat gatccaacct tcgggaagga aagaattttg cttaagat            48

<210>  65
<211>  45
<212>  DNA
<213>  Artificial Sequence

<220>
<223>  E13-5

<400>  65
aggtcaggat caacgctcaa gtgtctgttg cattgcttgt gttta                    45


<210>  66
<211>  46
<212>  DNA
<213>  Artificial Sequence

<220>
<223>  E13-3

<400>  66
catcttgcat gatccaacct tccgcacatt tctcatgttg tagctt                   46


<210>  67
<211>  45
<212>  DNA
<213>  Artificial Sequence

<220>
<223>  E14-5

<400>  67
aggtcaggat caacgctcaa gtttgccagt catttctgat ctctc                    45


<210>  68
<211>  47
<212>  DNA
<213>  Artificial Sequence

<220>
<223>  E14-3

<400>  68
catcttgcat gatccaacct tccaaagtgt ttgttccaat acagcag                  47


<210>  69
<211>  49
<212>  DNA
<213>  Artificial Sequence

<220>
<223>  E15-5

<400>  69
aggtcaggat caacgctcaa ggaggaagaa atctagaaaa caatcacag                49


<210>  70
<211>  48
<212>  DNA
<213>  Artificial Sequence

<220>
<223>  E15-3

<400>  70
catcttgcat gatccaacct tctggggaat aaaactatac ctacttgc                    48


<210>  71
<211>  46
<212>  DNA
<213>  Artificial Sequence

<220>
<223>  E16-5

<400>  71
aggtcaggat caacgctcaa gccagaaaac accacatcac tttaac                      46


<210>  72
<211>  48
<212>  DNA
<213>  Artificial Sequence

<220>
<223>  E16-3

<400>  72
catcttgcat gatccaacct tctgcaaggt attctgtaaa ggttcttg                    48


<210>  73
<211>  46
<212>  DNA
<213>  Artificial Sequence

<220>
<223>  E18-5

<400>  73
aggtcaggat caacgctcaa gagcacgttc ttctgctgta tgtaac                      46


<210>  74
<211>  47
<212>  DNA
<213>  Artificial Sequence

<220>
<223>  E18-3

<400>  74
catcttgcat gatccaacct tcatttgtgc attgttaagg aaagtgg                     47


<210>  75
<211>  43
<212>  DNA
<213>  Artificial Sequence

<220>
<223>  E19-5

<400>  75

aggtcaggat caacgctcaa ggaggtttca ccatgttggt cag                    43

<210>  76
<211>  47
<212>  DNA
<213>  Artificial Sequence

<220>
<223>  E19-3

<400>  76
catcttgcat gatccaacct tctaaagtcc cagctcttcc acttatc                47

<210>  77
<211>  45
<212>  DNA
<213>  Artificial Sequence

<220>
<223>  E20-5

<400>  77
aggtcaggat caacgctcaa gtccctctct cttcctctct tcttc                  45

<210>  78
<211>  42
<212>  DNA
<213>  Artificial Sequence

<220>
<223>  E20-3

<400>  78
catcttgcat gatccaacct tcctggaact ctggggttct cc                     42

<210>  79
<211>  45
<212>  DNA
<213>  Artificial Sequence

<220>
<223>  E21-5

<400>  79
aggtcaggat caacgctcaa gcagtggcaa attgacttaa aatcc                  45

<210>  80
<211>  45
<212>  DNA
<213>  Artificial Sequence

<220>
<223>  E21-3

<400>  80
catcttgcat gatccaacct tcggagtctt ttggcacagg tatgt                  45

<210>  81
<211>  45

<212>  DNA
<213>  Artificial Sequence

<220>
<223>  e22-5

<400>  81
aggtcaggat caacgctcaa gtttgacact ttgaatgctc tttcc                    45


<210>  82
<211>  43
<212>  DNA
<213>  Artificial Sequence

<220>
<223>  E22-3

<400>  82
catcttgcat gatccaacct tcattgtcct ctgtccaggc atc                      43


<210>  83
<211>  48
<212>  DNA
<213>  Artificial Sequence

<220>
<223>  E23-5

<400>  83
aggtcaggat caacgctcaa gaatcctaag aactcataca accaggac                 48


<210>  84
<211>  47
<212>  DNA
<213>  Artificial Sequence

<220>
<223>  E23-3

<400>  84
catcttgcat gatccaacct tcgtagagtg ctacactgtc caacacc                  47

## Description

### TECHNICAL FIELD

The present invention relates to the field of genetic diagnosis and genetic analysis of hereditary diseases and, more particularly, to methods for detection of rearrangements in a nucleic acid sequence.

### BACKGROUND ART

Large rearrangements are important DNA sequence alterations that involve the loss or gain of large fragments of DNA (ranging for 100 to 100,000 base pairs, bps) and usually cause important gene and gene product function variations. Many inherited diseases and cancer types are caused by large rearrangements in many genes (Human Gene Mutation Database, Human Genome Variation Society). These mutations can cause between 1 and 100% of the cases of many hereditary diseases, but more often they cause between 1 and 30%. More than 4.000 hereditary diseases are considered to involve large mutations or large rearrangements.

Cytogenetic abnormalities (CAs), such as trisomies, monosomies, deletions of chromosomal regions, and so on, are mutations that can lead to the loss or gain of genomic material and that are of great importance in the prenatal and postnatal genetic diagnosis. In addition, a special type of polymorphism called CNVs (copy number variations) is also caused by these losses or gains of DNA, but do not have a significant or known effect in the disease development.

Due to the increasing importance of the studies for genetic diagnosis, the possibility of determining the presence of large rearrangements in many genes to prevent wrong diagnosis of a significant percentage of patients is becoming more necessary.

Traditionally, large rearrangements have been studied by Southern blot. This technique requires a high amount of DNA, is laborious, time-consuming, and does not detect all rearrangements many times. The second method used is long PCR, which employs a low DNA amount and is faster, but is unable to detect some rearrangements (those involving more than 10-15 kb or insertions larger than 5.0 kb). Furthermore, small deletions or duplications involving hundreds of bases can be omitted when comparing very long fragments. However, it is a good option when screening for a particular known deletion or insertion. Recently, quantitative methods using fluorescence have been described. Real-time PCR methods have been used to detect large rearrangements in the BRCA1 gene and Rett Syndrome (Barrois et al. 2004 Clin Genet 65(2): 131-136; Ariani et al. 2004 Hum Mutat 24(2): 172-177). A procedure to detect large low density lipoprotein receptor (LDLR) gene rearrangements based on real-time PCR has been designed by Cantafora (Cantafora et al. 2004 Electrophoresis 25(21-22): 3882-3889). Other methods are based on DNA ligation and semiquantitative PCR, such as multiple ligation probe amplification (MLPA) (Schouten et al. 2002 Nucleic Acids Res 30 e57). A method known as universal primer quantitative fluorescent multiplex PCR (UPQFM-PCR), has been described to detect large rearrangements (Heath KE 2000 J Med Genet 37(4): 272-280). It consists of two consecutive PCR reactions, the first one with specific primers with an universal tail and the second one with the universally-labeled primers. These authors have set up the conditions to analyze five LDLR gene exons in the same procedure, and have shown how the method discriminates between wild-type and mutated alleles. Furthermore, the method can detect insertions and deletions as small as one base in the exons used in the screening. However, this method does not allow detecting all large rearrangements, since it only uses five exons (of the 18 of the LDLR gene) and the ratio between exons to identify the deleted/inserted fragments. Despite further improvement of this method, it maintained the need of two consecutive PCRs and DNA sequencing for fragment analysis (Garcia-Garcia et al. 2006 Hum Mutat 27(8): 822-8).

Detection of genetic alterations involving the loss or duplication of large genomic regions cannot be performed by conventional techniques such as PCR or long PCR. Common methods for detection of large rearrangements include Southern blotting and Multiplex Ligation-dependent Probe Amplification (MLPA). Southern blotting is a long, laborious and difficult to standardize technique when applied to large rearrangements detection, since a specific protocol is needed for each gene to be analysed for the presence of large rearrangements. The MLPA system is based on specific ligation of probes to different regions of a gene, semiquantitative PCR amplification, automatic sequencing and peak quantification. This system has greatly simplified the detection of such mutations, although it is still a long, laborious and expensive process, not indicated for diagnosis.

Cytogenetic abnormalities are usually detected by karyotyping, which involves cell culture and study of all chromosomes. One limitation of this technique is the difficulty in determining the presence of alterations involving regions smaller than 500,000 pairs of bases (bps). This problem is partially solved by the use of microarray CGH (comparative genomic hybridization). Another usual technique is Fluorescence in situ Hybridization (FISH), which detects and localizes the presence or absence of specific DNA sequences on chromosomes by fluorescent probes. There are also commercially available kits for the detection of major cytogenetic diseases such as duplication or loss of a chromosome (trisomies of 13, 18 and 21 chromosomes, as well as alterations of chromosomes X and Y). In the last years, analysis of genome by microarrays of single nucleotide polymorphisms (SNPs) and by specific probes has allowed the detection of many large alterations along genome, but these systems are expensive and time-consuming and they are not recommended to analyze specific regions since they analyze all genome. CNVs are usually analyzed by these kind of microchips. There is also the possibility of analyzing individual CNVs by kits commercially available.

Thus, there is a need for the development of alternative methods for analysis and/or detection of large rearrangements, cytogenetic abnormalities and polymorphisms such as copy number variations (CNVs), these alternative methods being easier to perform and able to overcome the problems associated to the methods described to date in the art.

### BRIEF SUMMARY OF THE INVENTION

In a first aspect, the invention relates to a PCR-based method for the detection of a rearrangement in a nucleic acid in a sample from a subject, wherein each PCR primer comprises two regions:
- a first region, which is not complementary to the nucleic acid target region to be analyzed and amplified, located at the 5' end of the PCR primer, and
- a second region, which is complementary to the nucleic acid target region to be analyzed and amplified, located at the 3' end of the PCR primer,
wherein the amplification product size and/or quantity of the nucleic acid target region to be analyzed and amplified, in the absence of a rearrangement, is determined and considered as the reference value, and
wherein if the amplification product size and/or quantity of the nucleic acid target region to be analyzed and amplified in said sample is substantially modified when compared to the reference value, then it is indicative of the presence of a gene rearrangement in the nucleic acid in said sample.

In a second aspect, the invention relates to a kit comprising a reagent adequate for detection of rearrangements in a nucleic acid sequence, wherein said reagent comprises PCR primers with two regions:
- a first region, which is not complementary to the nucleic acid target sequence to be analyzed for rearrangements, located at the 5' end of the PCR primer, and
- a second region, which is complementary to the nucleic acid target sequence to be analyzed for rearrangements, located at the 3' end of the PCR primer.

In a third and final aspect, the present invention relates to the use of a kit as previously mentioned in the diagnosis of diseases and disorders involving gene rearrangement

### BRIEF DESCRIPTION OF THE FIGURES

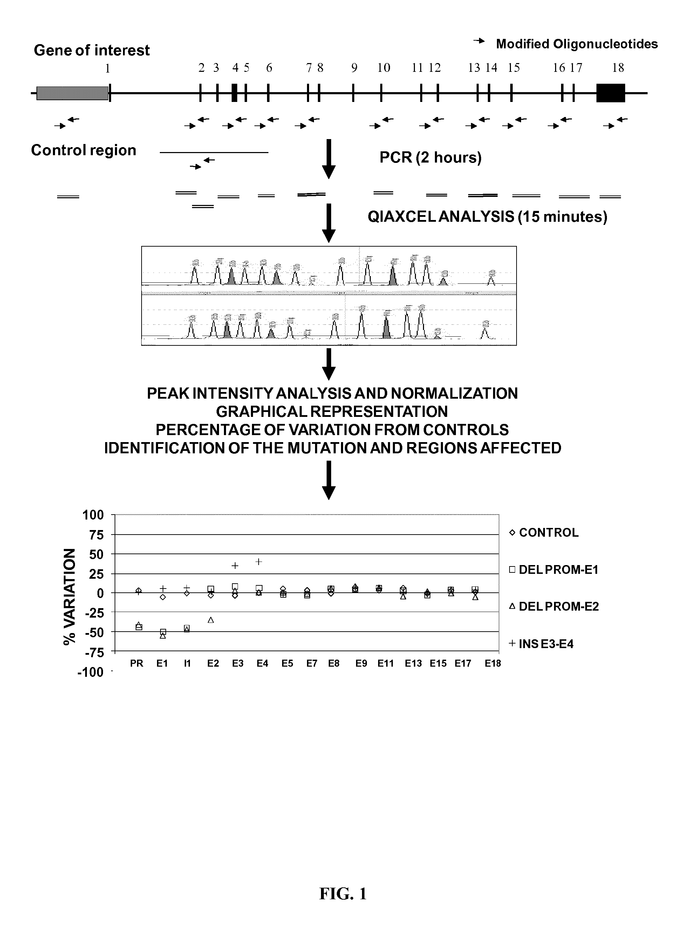

**Figure 1** shows a scheme for the working procedure according to the method of the invention. PCR products analysis is done by the QIAexcel system, involving peak intensity analysis and normalization, graphic representation, calculation of the percentage of variation compared to controls and identification of the mutation and affected regions.

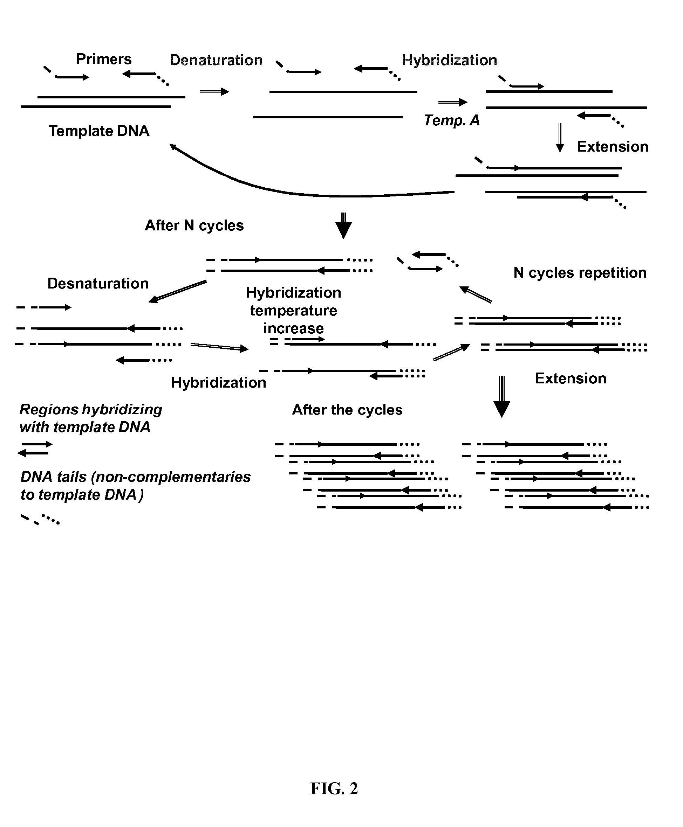

**Figure 2** shows the binding of the PCR primers to the template DNA along the two rounds of PCR amplification cycles. In the first round of PCR amplification cycles, the temperature for primer hybridization is set as the optimal temperature for hybridization of the primer sequence that is complementary to the template DNA. After this round, template DNA is proportionally amplified according to the initial DNA quantity for each particular DNA fragment. In the second round of PCR amplification cycles, hybridization temperature is increased so as the whole primer sequence can hybridize. This way, a more specific amplification is achieved, since amplification becomes less dependent on genomic sequences. After this second round of amplification cycles, a great number of copies is obtained, in proportion to the template DNA for each amplified fragment.

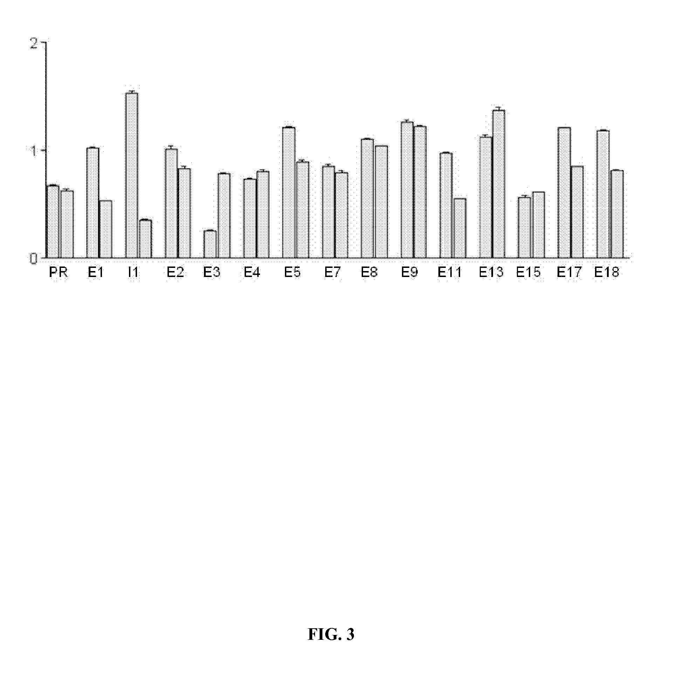

**Figure 3** shows a comparison between the results obtained using unmodified (left column of each amplified region) and modified (right column of each amplified region) oligonucleotides. Variation of the amplification intensity between fragments is greater when unmodified oligonucleotides are used. Amplification has been performed under optimal conditions for each of the oligonucleotide tested. Y axis indicates the ratio between amplicons of interest and control amplicon. Fragment intensities are normalized to an amplicon from a different gene known not to have any large rearrangement in the patients analyzed. PR: promoter region; E1: exon 1; I1: intron 1; E2: exon 2; E3: exon 3; E4: exon 4; E5: exon 5; E7: exon 7; E8: exon 8; E9: exon 9; E11: exon 11; E13: exon 13; E15: exon 15; E17: exon 17; E18: exon 18, all of them from LDLR gene.

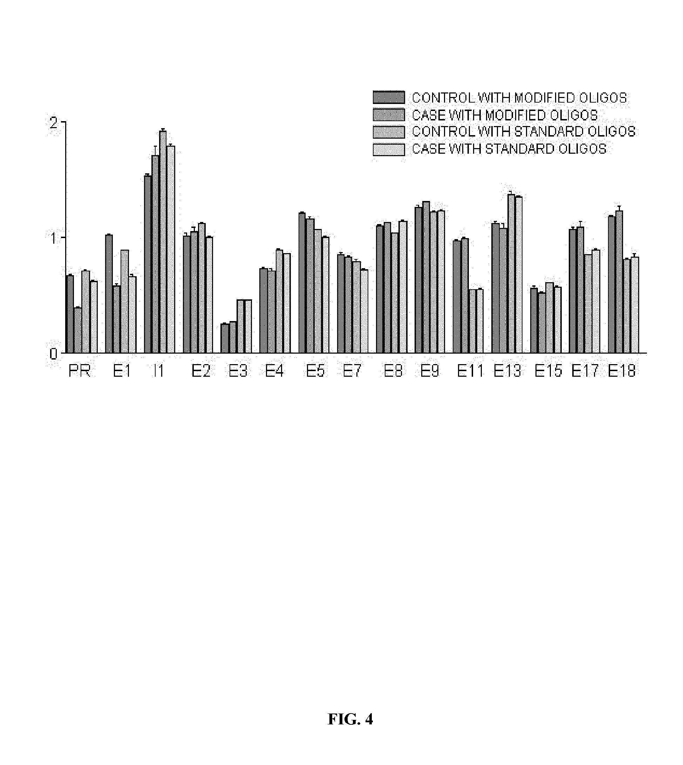

**Figure 4** shows a comparison of the discrimination capacity between non modified and modified oligonucleotides. The case shown corresponds to a deletion affecting promoter and exon 1. The differences in the intensities show that modified oligonucleotides allow improving the detection of this alteration. Amplification has been performed under optimal conditions for each of the oligonucleotide tested. Y axis indicates the ratio between amplicons of interest and control amplicons. PR: promoter region; E1: exon 1; I1: intron 1; E2: exon 2; E3: exon 3; E4: exon 4; E5: exon 5; E7: exon 7; E8: exon 8; E9: exon 9; E11: exon 11; E13: exon 13; E15: exon 15; E17: exon 17; E18: exon 18, all of them from LDLR gene.

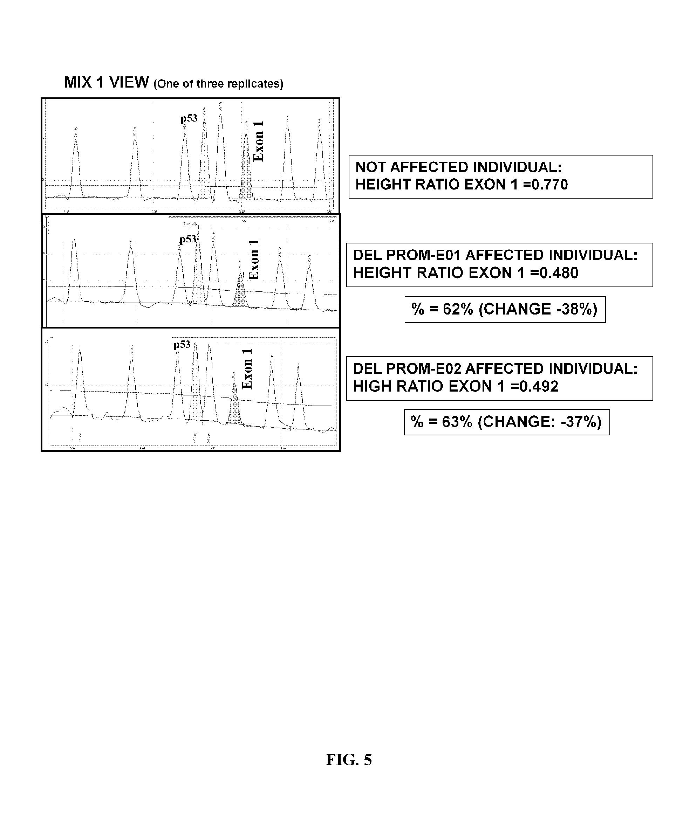

**Figure 5** shows the reproducibility of the fragment amplification and large rearrangement detection. Analysis of 8 fragments of LDLR gene for three samples. The first sample corresponds to a control sample from a non-affected individual. The second and third samples correspond to subjects having two different deletions affecting exon 1. Control fragment is indicated as p53 and marked with light grey. Exon 1, which is marked in grey, corresponds to the fragment with altered intensity.

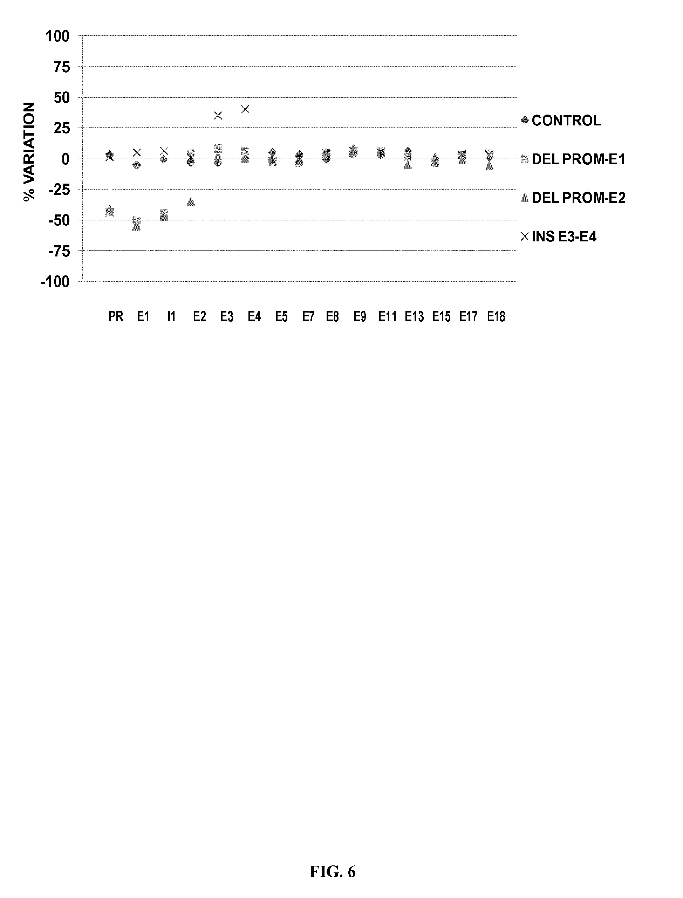

**Figure 6** shows interpretation of the electrophoresis of PCR products for LDLR gene (problem amplicons plus control amplicon) in relation to control samples. Analysis of a control sample without rearrangements in the gene of interest (diamonds), a sample from one patient with a deletion affecting promoter, exon1 and intron 1 regions (squares), a sample from one patient with a deletion affecting promoter, exon1 and exon 2 (triangles) and a sample from a patient with an insertion affecting exons 3 and 4 (crosses).

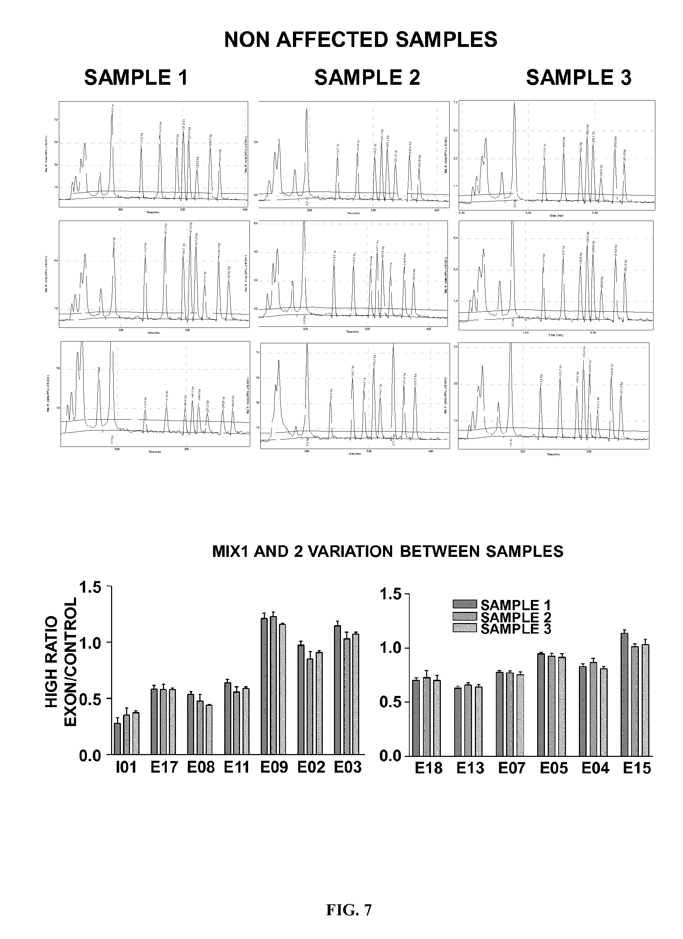

**Figure 7** shows the replication of results using the pools of initial oligonucleotides in three different experiments with 3 different samples.

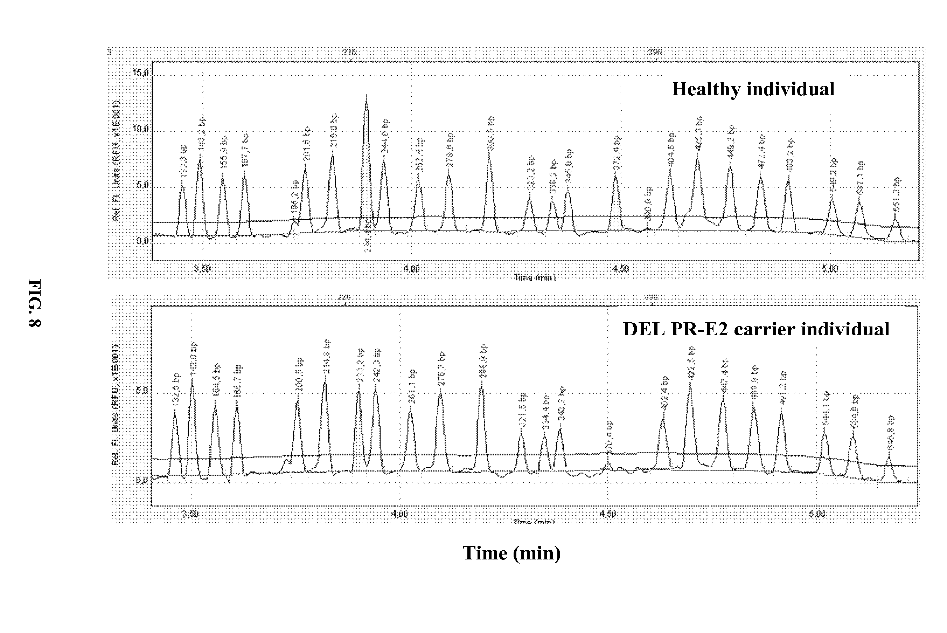

**Figure 8** shows the analysis for BRCA1 gene. Comparison of peak intensities between a healthy individual without rearrangement and a patient with an alteration (deletion of promoter region to exon 2) is shown. Peaks corresponding to Exon 1 and 2 amplicons are indicated in grey, for which a reduction of intensity is observed.

### DETAILED DESCRIPTION OF THE INVENTION

### Definitions

The terms "5'-end" and "3'-end" are used herein to indicate directionality in a strand of a nucleic acid. The term "5'-end" relates to the end of a nucleic acid strand that has the fifth carbon in the sugar-ring of the deoxyribose or ribose at its terminus. The term "3'-end" relates to the end of a nucleic acid strand that has a hydroxyl group at the third carbon of the sugar ring. Related terms "upstream" and "downstream" are used to indicate, respectively, towards the 5'-end and towards the 3'-end.

The term "allele" relates to one of two or more forms of a gene, locus or genetic polymorphism. Sometimes, different alleles can result in different traits; however, other times, different alleles will have the same result in the expression of a gene. Most multicellular organisms have two sets of chromosomes, that is, they are diploid. These chromosomes are referred to as homologous chromosomes. Diploid organisms have one copy of each gene (and one allele) on each chromosome. If both alleles are the same, they are homozygotes. If the alleles are different, they are heterozygotes.

The term "amplification" as used herein, is referred to the result of any method used to increase the number of copies of a nucleic acid molecule. The resulting products can be referred to as "amplicons" or "amplification products". Methods for amplifying nucleic acid molecules are known in the art, and include, but are not limited to, polymerase chain reaction (PCR), multiple displacement amplification (MDA), ligase chain reaction (LCR), Q-replicase amplification, polymerase chain reaction (PCR), degenerate oligonucleotide primed-polymerase chain reaction (DOP-PCR), rolling circle amplification (RCA), T7/primase-dependent amplification, strand-displacement amplification (SDA), self-sustained sequence replication (3SR), nucleic acid sequence-based amplification (NASBA) and loop-mediated isothermal amplification (LAMP).

The term "annealing temperature" relates to the temperature at which complementary regions of nucleic acids (generally monocatenary). In the context of a PCR reaction, the annealing temperature is the temperature at which oligonucleotides anneal to the single-stranded DNA template. In some embodiments, the annealing temperature is about 3-5 degrees Celsius below the temperature of melting (Tm) of the oligonucleotides. Stable DNA-DNA hydrogen bonds are only formed when the oligonucleotide sequence very closely matches the template sequence.

The term "binding region" is a segment or portion of a target nucleic acid molecule that is unique to the target molecule, and in some examples is free or substantially free of repetitive (or other undesired) nucleic acid sequence. The nucleic acid sequence of a binding region and its corresponding target nucleic acid molecule have sufficient nucleic acid sequence complementarity such that when the two are incubated under appropriate hybridization conditions, the two molecules will hybridize to form a detectable complex. A target nucleic acid molecule can contain multiple different binding regions, such as at least 10, at least 50, at least 100, or at least 1000 unique binding regions. In particular examples, a binding region is typically several hundred to several thousand base pairs in length. However, in some examples a binding region is shorter, such as 50 to 200 base pairs in length. When obtaining binding regions from a target nucleic acid sequence, the target sequence can be obtained in its native form in a cell, such as a mammalian cell, or in a cloned form (e.g., in a vector).

The term "binding or stable binding" refers to the association between two substances or molecules, such as the hybridization of one nucleic acid molecule (e.g., a binding region) to another (or itself) (e.g., a target nucleic acid molecule). Binding can be detected by any procedure known to one skilled in the art. Physical methods of detecting the binding of complementary strands of nucleic acid molecules include, but are not limited to, such methods as DNase I or chemical footprinting, gel shift and affinity cleavage assays, Northern blotting, dot blotting and light absorption detection procedures. In another example, the method involves detecting a signal, such as a detectable label, present on one or both nucleic acid molecules.

The term "chromosome rearrangement" relates to a kind of mutation involving a change in the normal arrangement of the genome into chromosomes. It occurs as a consequence of double-strand breaks of the DNA, followed by abnormal rejoining of the non-homologous ends, producing a new chromosomal arrangement. Alternatively, a chromosome rearrangement can result from crossing-over between repetitive DNA sequences. Usually, but not exclusively, this term applies to those changes that are visible cytogenetically. Chromosome rearrangements include, but are not limited to, deletions, duplications, insertions, inversions, translocations, and transpositions. The term "deletion" relates to a type of mutation caused by loss of one or more nucleotides from a DNA segment. Deletions can be very large, encompassing many genes and megabases of DNA, to the point of producing a visible cytological abnormality in a chromosome. Small deletions within a gene can alter the reading frame, and thus the amino acid sequence of the encoded protein. The term "duplication" relates to an additional copy of a DNA segment present in the genome. Duplications can lead to an increase in the number of genes carried on a chromosome and may or may not be cytologically visible. The term "insertion" relates to a type of mutation in which one or more nucleotides is inserted into a DNA sequence. Small insertions within a gene can alter the reading frame, and thus the amino acid sequence of the encoded protein. The term "inversion" relates to a type of mutation in which a length of DNA is broken in two positions and repaired in such a way that the medial segment is now present in reverse order. Inversions range in size from those large enough to be visible cytogenetically to those involving only a few base pairs, although there are inversions in smaller sequences as genes. The term "translocation" relates to an abnormality in which a part of a chromosome is broken and reattached to another part of the same chromosome or another chromosome and, hence, a change occurs in the shape of the chromosome. The term "transposition" relates to the movement or copying of a polynucleotide (transposon) from one location (donor site) to another (target site) often within or between a genome or genomes, or DNA constructs such as plasmids, bacmids, and cosmids. A database of chromosomal rearrangements in diseases (dbCRID) is available (Kong F et al. 2010 Nucleic Acids Res 39: D895-D900).

The term "CNV" or "copy number variation" is referred to a particular type of polymorphism characterized by an abnormal number of copies of one or more sections of the DNA. This term comprises both deletion of a relatively large genome regions and duplication of relatively large genome regions on certain chromosome regions. Each variation may range from about one kilobase to several megabases in size. In a particular embodiment of the invention, CNVs detected by the method of the invention comprise regions of at least 100 pb.

A nucleic acid molecule is said to be "complementary" with another nucleic acid molecule if the two molecules share a sufficient number of complementary nucleotides to form a stable duplex or triplex when the strands bind (hybridize) to each other, for example by forming Watson-Crick, Hoogsteen or reverse Hoogsteen base pairs. Stable binding occurs when a nucleic acid molecule remains detectably bound to a target nucleic acid sequence (e.g., genomic target nucleic acid sequence) under the required conditions. "Complementarity" is the degree to which bases in one nucleic acid molecule base pair with the bases in a second nucleic acid molecule (e.g., genomic target nucleic acid sequence). Complementarity is conveniently described by percentage, that is, the proportion of nucleotides that form base pairs between two molecules or within a specific region or domain of two molecules. For example, if 10 nucleotides of a 15 contiguous nucleotide region of a nucleic acid molecule form base pairs with a target nucleic acid molecule, that region of the nucleic acid molecule is said to have 66.67% complementarity to the target nucleic acid molecule. The term "sufficient complementarity" means that a sufficient number of base pairs exist between one nucleic acid molecule or region thereof and a target nucleic acid sequence (e.g., genomic target nucleic acid sequence) to achieve detectable binding. A thorough treatment of the qualitative and quantitative considerations involved in establishing binding conditions is provided by Beltz et al. Methods Enzymol. 100:266-285, 1983, and by Sambrook et al. (ed.), Molecular Cloning: A Laboratory Manual, 2nd ed., vol. 1-3, Cold Spring Harbor Laboratory Press, Cold Spring Harbor, NY, 1989. The term "non-complementarity" means that the number of base pairs between one nucleic acid molecule or region thereof and a target nucleic acid sequence in not sufficient to achieve binding between said two nucleic acids sequences. The term "non-complementary" is referred to nucleic acid molecules that do not share a sufficient number of complimentary nucleotides to form a stable duplex or triplex. The term "corresponding" in reference to a first and second nucleic acid (for example, a binding region and a target nucleic acid sequence) indicates that the first and second nucleic acid share substantial sequence identity or complementarity over at least a portion of the total sequence of the first and/or second nucleic acid. Thus, a binding region corresponds to a target nucleic acid sequence if the binding region possesses substantial sequence identity or complementarity (e.g., reverse complementarity) with (e.g., if it is at least 80%, at least 85%, at least 90%, at least 95%, or even 100% identical or complementary to) at least a portion of the target nucleic acid sequence. For example, a binding region can correspond to a target nucleic acid sequence if the binding region possesses substantial sequence identity to one strand of a double-stranded target nucleic acid sequence (e.g., genomic target DNA sequence) or if the binding region is substantially complementary to a single-stranded target nucleic acid sequence (e.g. RNA or an RNA viral genome).

The term "cycle" or "amplification cycle" or "PCR cycle" or "PCR amplification cycle" relates to a series of temperature changes involving discrete temperature steps in order to allow nucleic acids denaturation, annealing and extension during the PCR. In a particular embodiment, the PCR cycle involves two discrete temperature steps, the first temperature step for nucleic acid denaturation and the second temperature step for nucleic acid annealing and extension. In another particular embodiment, the PCR cycle involves three discrete temperature steps, the first step for denaturation, the second step for annealing and the third step for extension. Thus, a PCR amplification cycle involves a succession of a denaturing step, an annealing step and an elongation step.

The term "cytogenetic abnormality" is related to an alteration that affects chromosomes and it includes, but is not limited to, monosomies, trisomies and deletion of chromosomal regions. The term "monosomy" relates to a form of aneuploidy with the presence of only one chromosome from a pair. Partial monosomy occurs when only a portion of the chromosome has one copy, while the rest has two copies. Human conditions related to monosomy comprise, but are not limited to, Turner syndrome (women with one X chromosome instead of two X chromosomes), *cri du chat* syndrome (a partial monosomy caused by a deletion of the end of the short p arm of chromosome 5) and 1p36 deletion syndrome (a partial monosomy caused by a deletion at the end of the short p arm of chromosome 1). The term "trisomy" relates to a type of polysomy in which there are three copies, instead of the normal two, of a particular chromosome. Trisomies in humans comprise, but are not limited to, Down syndrome (trisomy 21), Edwards syndrome (trisomy 18), Patau syndrome (trisomy 13), trisomy 9, Warkany syndrome 2 (trisomy 8), trisomy 22, triple X syndrome (XXX), Klinefelter's syndrome (XXY), XYY. The term "deletion", in the context of a cytogenetic abnormality, is related to a portion of a chromosome that is missing or deleted. Human conditions related to deletion of chromosomal regions include, but are not limited to, Wolf-Hirschhom syndrome, which is caused by partial deletion of the short arm of chromosome 4, and Jacobsen syndrome (also called the terminal 11q deletion disorder).

The term "DNA polymerase" is referred to an enzymatic polypeptide that synthesizes new DNA strands by the incorporation of deoxyribonucleotides or analogs of deoxyribonucleotides to a nucleic acid polymer in a template-dependent manner.

The term "heterozygous" is referred to a diploid organism in which copies of a given gene have different alleles. A cell is said to be heterozygous for a particular gene when different alleles of a gene are present in homologous chromosomes. The cell or organism is called heterozygote.

The term "homozygous" is referred to a diploid organism in which both copies of a given gene have the same allele. A cell is said to be homozygous for a particular gene when identical alleles of the gene are present on both homologous chromosomes. The cell or organism in question is called a homozygote.

The term "melting temperature", "temperature of melting" or "Tm" is a quantitative expression of the stability of a hybrid of oligonucleotides, and is defined as the temperature at which a population of double-stranded nucleic acid molecules becomes half dissociated into single strands. In particular, when referred to oligonucleotides, it is referred to the temperature at which half of the oligonucleotides will form duplex with complementary oligonucleotides at the same concentration.

The term "nucleic acid" relates to a deoxyribonucleotide or ribonucleotide polymer in either single or double stranded form and, unless otherwise limited, encompasses natural nucleotides and analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in a polynucleotide. A "nucleotide sequence" or "nucleic acid sequence" refers to the sequence of bases in a polynucleotide.

The term "PCR" or "Polymerase Chain Reaction" relates to a DNA amplification process by means of polymerase chain reaction, that allows amplifying a single or a few copies of a DNA sequence across several orders of magnitude, generating as a result thousand to millions of copies of a particular DNA sequence. The PCR method relies on thermal cycling, consisting of cycles of repeated heating and cooling of the reaction for DNA melting and enzymatic replication of the DNA. Primers (short DNA fragments) containing sequences complementary to the target region along with a DNA polymerase (after which the method is named) are key components to enable selective and repeated amplification. As PCR progresses, the DNA generated is itself used as a template for replication, setting in motion a chain reaction in which the DNA template is exponentially amplified. PCR can be extensively modified to perform a wide array of genetic manipulations. The PCR process is well known in the art and is thus not described in detail herein. For a review of PCR methods and protocols, see, e.g., Innis et al, eds., PCR Protocols, A Guide to Methods and Application, Academic Press, Inc., San Diego, Calif. 1990; U.S. Pat. No. 4,683,202 (Mullis); which are incorporated herein by reference in their entirety. PCR reagents and protocols are also available from commercial vendors, such as Roche Molecular Systems. PCR may be carried out as an automated process with a thermostable enzyme. In this process, the temperature of the reaction mixture is cycled through a denaturing region, a primer annealing region, and an extension reaction region automatically. Machines specifically adapted for this purpose are commercially available. The PCR-based method of the invention will be described later on in detail. The term "long PCR" is referred to a particular type of PCR designed to amplify longer targets than those amplified with conventional PCR, ranging from one thousand to several thousand pairs of bases (bp) long. The term "multiplex PCR" relates to a PCR wherein multiple primer pairs are used in a single PCR mixture and amplification of various regions (usually the same number of primer pairs)of interest is performed simultaneously, allowing the simultaneous analysis of multiple different target sites in a single reaction.

The term "primer" or "oligonucleotide primer" or "oligonucleotide" or "oligo" as used herein, refers to an oligonucleotide that acts to initiate synthesis of a complementary nucleic acid strand when placed under conditions in which synthesis of a primer extension product is induced, e.g., in the presence of nucleotides and a polymerization-inducing agent such as a DNA or RNA polymerase and at suitable temperature, pH, metal ion concentration, and salt concentration. Primers are generally of a length compatible with their use in synthesis of primer extension products, and can be in the range of between about 8 nucleotides and about 100 nucleotides (nt) in length, such as about 10 nt to about 75 nt, about 15 nt to about 60 nt, about 15 nt to about 40 nt, about 18 nt to about 30 nt, about 20 nt to about 40 nt, about 21 nt to about 50 nt, about 22 nt to about 45 nt, about 25 nt to about 40 nt, and so on, e.g., in the range of between about 18 nt and about 40 nt, between about 20 nt and about 35 nt, between about 21 and about 30 nt in length, inclusive, and any length between the stated ranges. Primers can be in the range of between about 10-50 nucleotides long, such as about 15-45, about 18-40, about 20-30, about 21-25 nt and so on, and any length between the stated ranges. In some embodiments, the primers are not more than about 10, 12, 15, 20, 21, 22, 23, 24, 25, 26, 27, 28, 29, 30, 35, 40, 45, 50, 55, 60, 65, or 70 nucleotides in length. In this context, the term "about" may be construed to mean 1, 2, 3, 4, 5, 6, 7, 8, 9, 10, 11, 12, 13, 14, 15, 16, 17, 18, 19 or 20 more nucleotides either 5' or 3' from either termini or from both termini. Primers are in many embodiments single-stranded for maximum efficiency in amplification, but may alternatively be double-stranded. If double-stranded, the primer is in many embodiments first treated to separate its strands before being used to prepare extension products. This denaturation step is typically effected by heat, but may alternatively be carried out using alkali, followed by neutralization. Thus, a primer is complementary to a template, and complexes by hydrogen bonding or hybridization with the template to give a primer/template complex for initiation of synthesis by a polymerase, which is extended by the covalent addition of bases at its 3' end. A "primer pair" as used herein refers to first and second primers having nucleic acid sequence suitable for nucleic acid-based amplification of a target nucleic acid. Such primer pairs generally include a first primer having a sequence that is the same or similar to that of a first portion of a target nucleic acid, and a second primer having a sequence that is complementary to a second portion of a target nucleic acid to provide for amplification of the target nucleic acid or a fragment thereof. Reference to "first" and "second" primers herein is arbitrary, unless specifically indicated otherwise. For example, the first primer can be designed as a "forward primer", also known as "sense primer" (which initiates nucleic acid synthesis from the 5' end of the target nucleic acid) or as a "reverse primer", also known as "antisense primer" (which initiates nucleic acid synthesis from the 5' end of the complementary strand of the target nucleic acid).

The term "reference value" in the context of the PCR-based method of the invention is related to the quantity and/or the size of a PCR amplification product obtained by amplification of a nucleic acid target region under analysis for a particular rearrangement from a sample known not to have said rearrangement.

The term "rearrangement" relates to a genomic alteration that involves the loss or gain of fragments of DNA. In a particular embodiment of the invention, the rearrangement affects a region comprising at least 1 base pair (bp), more preferably at least 100 bp. Rearrangements according to the invention can involve regions of 1 pb to several kpb, in particular regions of 1 pb to 10 kbp, more in particular 100 bp to 100 kpb, or even more. In a particular embodiment, the term "rearrangement involving an insertion" is related to any rearrangement wherein a gain of a DNA fragment occurs. In an alternative particular embodiment, the term "rearrangement involving a deletion" is related to any rearrangement wherein a loss of a DNA fragment occurs. The rearrangement may involve genomic alterations affecting one or more chromosomes.

The term "retrotranscription" or "reverse transcription" is related to a process of synthesis of a single-stranded DNA, known as cDNA or complementary DNA, from RNA such as mRNA as template. Methods for cDNA synthesis are known by the skilled person. Kits are commercially available as well including, without being limited to, SuperScript® VILO™ cDNA synthesis kit from Life Technologies, QuantiTect Rev. Transcription kit from Qiagen, Maxima First Strand cDNA synthesis kit from Thermo Scientific, iScript cDNA Synthesis kit from Bio-Rad, cDNA Synthesis kit from Invitrogen and GoScript™ Reverse Transcription System from Promega.

The term "sample" or "biological sample", as used in the context of the present invention, refers to any biological material that can be obtained from a living being (including, without being limited to, animals, plants, fungi and bacteria) is susceptible of preservation and can hold information of the genetic profile of the subject. Biological samples according to the invention include, without being limited to, blood, a biopsy sample, a tissue, a cell or a fluid (serum, saliva, semen, sputum, cerebral spinal fluid (CSF), tears, mucus, sweat, milk, brain extracts, cultured cells and the like). In a preferred embodiment of the invention, the sample is DNA obtained from whole blood or serum. The term "whole blood" means blood from a human or animal containing both cellular components and liquid component. Whole blood can be in coagulated state of non-coagulated state. "Whole blood" also includes blood wherein portion or all of the cellular components, such as white blood cells or red blood cells, have been lysed. The term "serum" refers to plasma without the clotting protein fibrinogen and other clotting factors. Samples according to the invention can be obtained by conventional methods using processes known in the state of the art by the person skilled, such as blood extraction or biopsy extraction. Methods for obtaining the sample from the biopsy include gross apportioning of a mass, or microdissection or other art-known cell-separation methods. Cells can additionally be obtained from fine needle aspiration cytology. In order to simplify conservation and handling of the samples, these can be formalin-fixed and paraffin-embedded or first frozen and then embedded in a cryosolidifiable medium, such as OCT-compound, through immersion in a highly cryogenic medium that allows for rapid freeze. The biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. DNA is then extracted from frozen or fresh samples by any of the methods typical in the art, for example, those disclosed in Sambrook, J., et al., 2001. Molecular cloning: A Laboratory Manual, 3rd ed., Cold Spring Harbor Laboratory Press, N.Y., Vol. 1-3. Samples can also be formalin-fixed, paraffin-embedded tissue samples.

The term "specifically hybridizing", as used herein, refers to the conditions which allow the hybridization of two polynucleotides under high stringent conditions or moderately stringent conditions. The "stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and the target sequence, the higher the relative temperature which must be used. As a result, it follows that higher relative temperatures would tend to make the reaction conditions more stringent, while lower temperatures less so. See Brown T, "Gene Cloning" (Chapman & Hall, London, UK, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, UK, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

The term "stringent conditions" or "high stringency conditions", as used herein, refers to: (1) the use low ionic strength solutions and high temperature for washing, such as for example, 0.015 M sodium chloride/0.0015 M sodium citrate/0.1% sodium dodecyl sulfate at 50° C.; (2) the use, during hybridization, of a denaturing agent, such as formamide, in a 50% (v/v) formamide solution with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) the use of 50% formamide solution, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C. The term "moderately stringent conditions", as used herein, refers to hybridization conditions known in the art, and may include the use of washing solution and hybridization conditions (e.g. temperature, ionic strength and % SDS) less stringent that those described before. See Sambrook, 1989, supra. An example of moderately stringent conditions is the overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, and other parameters as required for accommodating factors such as probe length and relative abundance of nucleotides.

The term "subject", as used herein, refers to an individual, such as a human, a nonhuman primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs; and other living beings including plants, fungi and bacteria. The term does not denote a particular age or sex. The term "subject" encompasses an embryo and a fetus. In a particular embodiment of the invention, the subject is a human.

The term "substantially modified" in the context of the method of the invention, refers to an increase or to a decrease in the size and/or the quantity of the amplification product to be analyzed and amplified by said method when compared to a reference value.

### PCR-based method of the invention

The authors of the present invention have developed a method useful for the detection of large rearrengements, cytogenetic abnormalities and polymorphisms such as copy number variations (CNVs) that involves a PCR wherein primers comprise a 5' end sequence non-complementary to the nucleic acid region to be analyzed and amplified, and wherein the size and/or the PCR amplification product is compared to a reference value to determine if an insertion or a deletion has occurred in the analyzed region. By this method, the inventors have analyzed rearrangements in the LDLR gene (see example 1, showing detection of insertions and deletions in the LDLR gene) and BRCA1 gene (see example 2, showing detection of deletion in the BRCA1 gene).

The method developed by the inventors, when applied to large rearrangements detection, has the great advantage of being much more rapid, sensitive and easy to analyze than Southern blotting or even the MLPA system, since it requires far fewer reaction steps and reagent handling. When applied to cytogenetic alterations analysis, the method described herein is much faster than karyotyping, FISH and microarrays when directed to specific chromosomal abnormalities or a group of them. Besides, the method of the invention is much easier to analyze and reliable than existing systems based on the study of polymorphic markers. Besides, when the method of the invention is applied to CNVs analysis, it has the advantage that it allows analyzing various points of the CNV or even analyzing several CNVs at the same time, in contrast to commercially available systems. Although the method of the present invention can be used in the detection of rearrangements in homozygosis, the method disclosed herein is especially interesting and advantageous over the methods in the art for the detection of rearrangements in heterozygosis.

Thus, in a first aspect, the invention is related to a PCR-based method for the detection of a rearrangement in a nucleic acid region in a sample from a subject, wherein each PCR primer comprises two regions:
- a first region which is not complementary to the nucleic acid target sequence to be analyzed and amplified, located at the 5' end of the PCR primer, and
- a second region which is complementary to the nucleic acid target sequence to be analyzed and amplified, located at the 3' end of the PCR primer,
wherein the amplification product size and/or quantity of the nucleic acid target region to be analyzed and amplified, in the absence of a rearrangement, is determined and considered as the reference value, and wherein if the amplification product size and/or quantity of the nucleic acid target region to be analyzed and amplified in said sample is substantially modified when compared to the reference value, then it is indicative of the presence of a gene rearrangement in the nucleic acid in said sample.

It is known for the skilled person that a basic PCR set up requires several components and reagents, comprising: a template nucleic acid, usually DNA, comprising the target region to be analyzed and amplified, at least a pair of primers, that are complementary to each of the sense and anti-sense strand of the target region, a DNA polymerase, deoxynucleoside triphosphates, a buffer solution, divalent cations and monovalent cations. Detection and analysis of the PCR amplification products is required as well by the method of the invention.

### Template nucleic acid

The template nucleic acid according to the PCR-based method of the invention is a DNA obtained from a biological sample wherein the detection of rearrangements is to be performed by the method of the invention or a control DNA from a control sample. The template nucleic acid comprises the target sequence to be analyzed and amplified by the PCR method of the invention, in order to determine if any rearrangement exists in said nucleic acid. The template is added to the amplification reaction as the starting material from which the amplification reaction proceed. The term "biological sample" has been previously defined in the description. Preferred biological samples according to the invention comprise blood and any sample comprising nucleated cells, more preferably the sample according to the invention is blood. Biological samples according to the invention comprise both samples from subjects under study and samples from control subjects. Control DNA samples can be obtained from subjects known not to have a rearrangement in the genomic target sequence under analysis. In a preferred embodiment, the reference value of the PCR-based method of the invention is derived from such a control DNA sample obtained from a subject known not to have the rearrangement under analysis. Alternatively, a control sample can also be obtained from a subject known to have a particular rearrangement in a genomic target sequence under analysis.

Methods to extract nucleic acids, such as methods to extract RNA or to extract DNA, from a biological sample are known in the art. A great number of commercially available kits for nucleic acid isolation are available including, but not limited to, Wizard® genomic DNA purification kit from Promega, QIAamp DNA kits from Qiagen, PureLink™ Genomic DNA purification kits from Invitrogen, Genomic DNA purification kits from Fermentas, RNeasy Mini kit from Qiaqen, TRIzol® Reagent, etc. In a particular embodiment of the invention, the template DNA in the PCR reaction comprises DNA isolated from a biological sample from a subject whose nucleic acid rearrangements are to be analyzed. In a preferred embodiment, the template DNA in the PCR reaction comprises DNA isolated from a biological sample from a subject whose nucleic rearrangements are to be analyzed and a control DNA.

A control DNA according to the invention is selected from the group including, but not limited to, DNA isolated from a biological sample from a subject known not to have a particular rearrangement for a particular target genomic region under study and DNA isolated from a biological sample from a subject known to have a particular rearrangement for a particular target genomic region. The control DNA derives from a sample or a sample collection formed preferably by a mixture of samples from normal subjects or from subjects not affected by the particular gene rearrangement under analysis.

Both DNA sample under analysis and DNA control sample may also comprise a DNA fragment comprising a genomic region which is different from the target genomic region under rearrangement analysis, for normalization of the amplification values obtained from the target genomic region under study. According to the invention, at least one control region is used, preferable between 1 and 10 control regions, more preferably between 1 and 4 control regions. The analysis of these control regions allows analyzing the results by lowering the effects due to differences in template DNA quantities used in each sample or PCR.

In a particular embodiment, the template nucleic acid for the PCR-based method of the invention is cDNA obtained by means of retrotranscription or reverse transcription. In a particular preferred embodiment, the template nucleic acid for the PCR-based method of the invention is DNA.

Target nucleic acid regions according to the present invention are those genomic regions known to be susceptible of rearrangement, wherein said rearrangements are related to diseases or disorders. Any gene or genomic region susceptible of having a rearrangement can be the target of the PCR-based method according to the present invention. A non exhaustive list of genes in which rearrangements have been related to hereditary diseases and disorders, and wherein rearrangements can be detected by the method of the invention include, without being limited to, ABCA1, ABCB11, ABCG5, ABCG8, ANK2, APOA1, APOB, APOC2, APOE, ARH, ATP8B1, BRCA1, BRCA2, CBS, CETP, CFTR, CLCNKB, CLDN16, CLDN19, COL1A1, COL1A2, COL3A1, DAX1, DMGDH, FBN1, FLCN, FMO1, FMO3, GCK, HNF1A, HNF1B, HNF4A, KCNE1, KCNE2, KCNH2, KCNJ2, KCNQ1, LCAT, LDLR, LPL, MEFV, Menin, MLH1, MSH2, MTHFR, MTP, NF1, NF2, PCSK9, PKP2, PRKAR1A, PRSS1, RET, SCN5A, SDHB, SDHC, SDHD, SERPINA1, SLC12A3, SLC22A1, SLC34A2, SPINK1, TGFBR1 and TGFBR2. In a more particular embodiment, the method according to the invention detects rearrangements in genes selected from the group comprising BRCA1 and LDLR.

### PCR primers

According to the PCR-based method of the invention, each PCR primer comprises two regions:
- a first region which is not complementary to the nucleic acid target sequence to be analyzed and amplified, located at the 5' end of the PCR primer, and
- a second region which is complementary to the nucleic acid target sequence to be analyzed and amplified, located at the 3' end of the PCR primer.

The first region of the PCR primers according to the invention can also be referred to as a "tail" or a "primer tail". According to the invention, both sense and antisense primers of the primer pair for the amplification of a particular region comprise such a first region or tail. The length of this first region of the primers is of at least 1 base, at least 2 bases, at least 3 bases, at least 4 bases, at least 5 bases, at least 6 bases, at least 7 bases, at least 8 bases, at least 9 bases, at least 10 bases, at least 12 bases, at least 15 bases, at least 20 bases, at least 30 bases or at least 50 bases. In a particular embodiment, the length of the first region of the primers is between 5 and 40 bases. In a more particular embodiment, the length of the first region of the primers is between 10 and 30 bases.

The length of the second region of the PCR primers is such that specific hybridization of this second region of the primer and the template occurs. The length of this second region of the primers is of at least 5 bases, at least 6 bases, at least 7 bases, at least 8 bases, at least 9 bases, at least 10 bases, at least 12 bases, at least 15 bases, at least 20 bases, at least 30 bases or at least 50 bases. In a particular embodiment, the length of the second region of the primer is between 10 and 40 bases. In a more particular embodiment, the length of the first region of the primers is between 12 and 35 bases.

The amplification and analysis of a target sequence involves a primer pair, i.e., a forward primer and a reverse primer. In a particular embodiment of the invention, the length of the forward primer tail is the same as the length of the reverser primer tail. In a particular embodiment both the length and the sequence of the forward primer tail and the reverse primer tail are the same. In a particular embodiment of the invention, the length of the forward primer tail is different from the length of the reverser primer tail. In a particular embodiment of the invention, the sequence of the forward primer tail is different from the sequence of the reverser primer tail. In a particular embodiment of the invention, the sequence and the length of the forward primer tail is different from the sequence and the length of the reverser primer tail. In a particular preferred embodiment of the invention, the length of the forward primer tail is the same as the length of the reverser primer tail and the sequence of the forward primer tail is different from the sequence of the reverser primer tail.

In a particular embodiment, the PCR-based method of the invention involves two primer pairs, a first primer pair for amplification of the region wherein the presence of a rearrangement is to be analyzed and a second primer pair for amplification of a control region for normalization, wherein the control region is a region known to be present in the nucleic acid sample under analysis for the presence of a rearrangement and results in an amplicon whose size is known. In a preferred embodiment of the invention, the PCR-based method of the invention is a multiplex PCR that involves at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 primer pairs, at least 25, at least 30, at least 35, at least 40, at least 45 or at least 50, wherein at least one primer pair is for amplification of a control region for amplification normalization. In a preferred embodiment of the invention, the multiplex PCR-based method of the invention involves between 5 and 60 primer pairs. In a more preferred embodiment of the invention, the multiplex PCR-based method of the invention involves between 10 and 40 primer pairs.

The conditions to perform a multiplex PCR need to be optimized according to the particular target regions of interest being analyzed and amplified and the primer pairs to achieve such amplification. Thus, annealing temperatures of primers in a multiplex PCR must be optimized to function correctly within a single reaction. As the skilled person understands, the melting temperature of the primers in a multiplex PCR must be similar, so all the primers can hybridize to the template at the annealing temperature during the PCR. In particular, the differences between the melting temperatures of primers in a multiplex PCR are less than 1 °C, less than 2 °C, less than 3 °C, less than 4 °C, less than 10 °C or less than 14 °C. The annealing temperature of a primer depends directly on its length and composition, in particular its GC content. In general, multiplex PCR primers have a GC content between 30 and 70%, preferably between 40 and 65%, more preferably between 50 and 60%. Additionally, according to the PCR based method of the invention, primer pairs in the multiplex PCR produce amplicons of varying sizes which are specific to different target sequences. As discussed later, amplicons obtained from the multiplex PCR-based method of the invention must have such a length that allows later on their distinction by their different length.

In a particular embodiment of the invention, the multiplex PCR-based method involves a number of primer pairs wherein forward primer tails have the same length and sequence. In a particular embodiment of the invention, the multiplex PCR-based method involves a number of primer pairs wherein reverse primer tails have the same length and sequence. In a more particular embodiment of the invention, the multiplex PCR-based method involves a number of primer pairs wherein forward primer tails have the same length and sequence and reverse primer tails have the same length and sequence, wherein forward primer tails have different length and sequence from reverse primer tails.

The PCR primers according to the PCR-based method of the invention comprise two regions, as previously described, wherein a second region, located at the 3' end of the primer, is complementary to the nucleic acid region target region to be analyzed and amplified. In a particular embodiment of the invention, the PCR primers hybridize to regions of the nucleic acid template within the region under analysis. In a particular embodiment, both primers of the primer pair hybridize to regions of the nucleic acid template within the region under analysis. In a particular embodiment of the invention, the PCR primers hybridize to regions of the nucleic acid template flanking the rearrangement region under analysis. In a particular embodiment, both primers of the primer pair hybridize to regions of the nucleic acid template flanking the rearrangement region under analysis. In a particular alternative embodiment, one primer of the primer pair hybridizes to a region of the nucleic acid template flanking the rearrangement region under analysis and the other primer of the primer pair hybridize to a region of the nucleic acid template within the rearrangement region under analysis.

The PCR primers according to the PCR-based method of the invention may or may not be labeled with a detectable agent. In a particular embodiment, the oligonucleotide is conjugated to a detectable agent. In an alternative preferred embodiment, the oligonucleotide used is not conjugated to a detectable agent.

The terms "detectable agent" and "labeling" are synonyms and they refer to an agent the nature of which allows its detection by means of enzymatic, radioactive or fluorescence methods. The detectable compound can be an enzyme, a radioactively labeled compound or a radioactive isotope, a fluorochrome, a chemiluminescent reagent, an enzymatic substrate or cofactor, an enzymatic inhibitor, a particle, a dye, etc.

The compounds radioactively labeled by means of radioactive isotopes, also called radioisotopes or radionuclides, may include, without limitation, ³H, ¹⁴C, ¹⁵N, ³⁵S, ⁹⁰Y, ⁹⁹Tc, ¹¹¹In, ¹²⁵I, ¹³¹I. The fluorescent labels may include, without limitation, rhodamine, phosphorus-lanthanides or FITC. The enzymatic labels may include, without limitation, horseradish peroxidase, β-galactosidase, luciferase or alkaline phosphatase. The preferred labeling include, but are not limited to, fluorescein, a phosphatase such as alkaline phosphatase, biotin, avidin, a peroxidase such as horseradish peroxidase and compounds related to biotin or compounds related to avidin (for example, streptavidin or ImmunoPure® NeutrAvidin available from Pierce, Rockford, IL).

PCR primer design can be performed by strategies known by the skilled person. Primers can be selected manually, or with the assistance of a computer implemented algorithm that optimizes primer selection based on parameters known by the skilled person, such as annealing temperature, length, GC content, etc. Numerous computer implemented algorithm or programs for use via the internet or on a personal computer are available including, without being limited to, those at or from the indicated web address. A non-exclusive list of such programs (with corresponding Internet addresses) includes: CODEHOP (blocks.fhcrc.org/codehop.html); Gene Fisher (bibiserv.techfak.uni-bielefeld.de/genefisher/); DoPrimer (doprimer.interactiva.de/); Primer3 (genome.wi.mit.edu/cgi-bin/primer/primer3_www.cgi); Primer Selection (alces.med.umn.edu/rawprimer.html); Web Primer (genome-www2.stanford.edu/cgi-bin/SGD/web-primer); PCR Designer (cedar.genetics.soton.ac.uk/public_html/primer.html); Primo Pro 3.4 (changbioscience.com/primo/primo.html); Primo Degenerate 3.4 (changbioscience.com/primo/prmod.html); PCR Primer Design (pga.mgh.harvard.edu/servlet/org.mgh.proteome.Primer); The Primer Generator (med.jhu.edu/medcenter/primer/primer.cgi); EPRIMER3 (bioweb.pasteur.fr./seqanal/interfaces/eprimer3.htm13); PRIMO (bioweb.pasteur.fr/seqanal/interfaces/primo.html3); PrimerQuest (idtdna.com/biotools/primer_quest/primer_quest.asp); MethPrimer (itsa.ucsf.edu/- urolab/methprimer/index1.html); Rawprimer (alces.med.umn.edu/rawprimer.html); MEDUSA (cgr.ki.se/cgr/MEDUSA); The Primer Prim'er Project (nmr.cabm.rutgers.edu/bioinformatics/Primer_Primer_Project/Primer.html); Oligonucleotides for the PCR (cit2.fr/bio2/Oligo2lib.html); GAP (promoter.ics.uci.edu/Primers/); Oligonucleotides pour la PCR (cit2.fr./bio2/OligoTM.html); Oligonucleotide properties calculator (asic.nwu.edu/biotools/oligocalc.html); Oligonucleotide analyzer (mature.com/oligonucleotide.html); Oligo Tm Determination (alces.med.umn.edu/rawtm.html); Poland (biophys.uniduesseldorf.de/local/POLAND/poland.html); PROLIGO (gensetoligos.com/Calculation/calculation.html); PrimerSelect (dnastar.com); DNASIS Max (medprobe.com/no/dnasis.html); Primer Premier 5 (premier/biosoft.com/); Primer Premier (premier/biosoft.com/); NetPrimer (piemierbiosoft.com/NetPrimer.html); Array Designer 2 (premierbiosoft.com/dnamicrorray-ray/dnamicroarray.html); Beacon Designer 2.1 (premierbiosoft.com/molecular_beacons/taqman_molecular_beacons.html); GenomePRIDE 1.0 (pride.molgen.mpg.de/genomepride.html); Fast PCR (biocenter.helsinki.fi/bi/bare-1_html/manual.htm); OLIGO 6 (oligo.net/); Primer Designer 4 (scied.com/ses_pd5.htm); GPRIME (life.anu.edu.au/molecular/software/gprimer.htm); Sarani Gold (mail.standgenomics.cm/products/sarani/); PCR Help (techne.com/CatMol/pcrhelp.htm); Genorama chip Design Software (asperbio.com/Chip_desian_soft.htm); Primer Designer (genamics.com/expression/primer.htm); Primer Premier (biotechniques.com/freesamples/itembtn21.html); and PrimerDesign (chemie.unimarbura.de/%7becker/pdhome.html).

### DNA polymerase

A DNA polymerase enzymatically assembles a new DNA strand from nucleotides, by using single-stranded DNA as a template and DNA primers (also referred to as DNA oligonucleotides), which are required for initiation of DNA synthesis.

DNA polymerizing enzymes can be thermostable (heat stable) polymerases or thermodegradable polymerases. Heat stable polymerases allow a polymerization reaction to be initiated or shut down by changing the temperature or other condition in the reaction mixture without destroying activity of the polymerase. Specific examples of thermostable polymerases illustratively include those isolated from *Thermus aquaticus, Thermus thermophilus, Pyrococcus woesei, Pyrococcus furiosus, Thermococcus litoralis* and *Thermotoga maritima.* Thermodegradable polymerases illustratively include *E. coli* DNA polymerase, the Klenow fragment of *E. coli* DNA polymerase, T4 DNA polymerase, T7 DNA polymerase and other examples known in the art. It is recognized in the art that other polymerizing enzymes are similarly suitable illustratively including E. coli, T7, T3, SP6 RNA polymerases and AMV, M-MLV, and HIV reverse transcriptases.

DNA polymerases according to the PCR-based method of the invention include, but are not limited to, Taq polymerase (originally isolated from the bacterium *Thermus aquaticus*), Pfu polymerase (*Pyrococcus furiosus*), Pwo polymerase (*Pyrococcus woesei*), Tfl polymerase (*Thermas flavus*), rTth polymerase (*Thermus thermophilus*), Tli polymerase (*Thermus litoris*) and Tma polymerase (*Thermotoga maritime*).

The PCR-based method of the invention comprises a DNA polymerase, more preferably a heat-stable or thermo stable DNA polymerase. In a particular embodiment, the DNA polymerase used in the PCR-based method of the invention has an optimum temperature of activity between 70 and 74 °C, preferably of 72 °C. In a preferred embodiment, the DNA polymerase used in the PCR-based method of the invention is selected from the group of thermostable DNA polymerases comprising, without being limited to, Taq DNA polymerase from *Thermus aquaticus*, Pfu DNA polymerase from *Pyrococcus furiosus* and Vent DNA polymerase from *Thermococcus litoralis,* KOD DNA polymerase from *Thermococcus kodakaraensis* and NovaTaq DNA polymerase from *Thermus aquaticus*.

### dNTPs, buffers, divalent cations, monovalent cations

Deoxynucleoside triphosphates (dNTPs) are nucleotides containing triphosphate groups and include dATP, dTTP, dCTP o dGTP, which differ in their nitrogenated base. dNTPs are usually present in the PCR reaction in equimolar proportions. Usual concentrations of dNTPs in the reaction mixture are known by the skilled person, usually ranging from 50 µM to 500 µM, usually 200 µM.

The PCR buffer solution provides a suitable chemical environment (pH, ionic strength, cofactors, etc.) for optimum activity and stability of the DNA polymerase. PCR buffers are commercially available and, usually, formulated specifically for each DNA polymerase. Non-limitative examples of commercially available PCR buffers include the *Taq* DNA polymerase PCR buffer from Invitrogen, *Taq* DNA polymerase ThermoPol buffer from New England Biolabs, GeneAmp PCR buffer I from Applied Biosystems and BloodDirect™ PCR buffer kit from Merck Millipore (for PCR amplification directly from human blood),

Common divalent cations used in PCR include, but are not limited to, magnesium (Mg²⁺) or manganese (Mn²⁺) ions.

Common monovalent cations used in PCR include, but are not limited to, K⁺.

### Amplification cycles of the PCR-based method of the invention

The PCR-based method of the invention involves a series of amplification cycles, wherein each amplification cycle involves denaturation, annealing and extension. In a particular embodiment, annealing and extension occur at the same temperature, which is different to the denaturation temperature. In another particular embodiment, annealing and extension occur at different temperatures, which are also different to the denaturation temperature.

In a particular embodiment, the PCR-based method of the invention comprises 15-50 amplification cycles, preferably 20-45 amplification cycles, more preferably 25-40 amplification cycles. The PCR according to the method of the invention is carried out in a reaction volume of 5-100 µl, preferably in a reaction volume of 5-20 µl, more preferably in a reaction volume of 10-15 µl. The PCR is carried out in small reaction tubes, usually of 40-100 µl volume, in a thermal cycler.

The PCR-based method of the invention involves thermal cycling, i.e., alternately heating and cooling the PCR sample to a defined series of temperature steps. These thermal cycling steps are necessary first to physically separate the two strands in a DNA double helix at a high temperature during the denaturation step. At a lower temperature, PCR primers bind to their complementary sequences and each strand is then used as the template in DNA synthesis by the DNA polymerase to selectively amplify the target DNA.

The thermal cycler heats and cools the reaction tubes to achieve the temperatures required at each step of the reaction. Many thermal cyclers make use of the Peltier effect, which permits both heating and cooling of the block holding the PCR tubes simply by reversing the electric current. Thin-walled reaction tubes permit favourable thermal conductivity to allow for rapid thermal equilibration. Most thermal cyclers have heated lids to prevent evaporation and condensation at the top of the reaction tube. Older thermocyclers lacking a heated lid require a layer of oil on top of the reaction mixture or a ball of wax inside the tube. Available thermocyclers to perform the PCR-based method of the invention include, but are not limited to, Swift™ MaxProThermal Cyclers (Esco Micro Pte Ltd), Biometra T3000 Thermocyclers (Labrepco), Dual 96-Well GeneAmp® PCR System 9700 (Life Technologies), Arktik Thermal Cyclers (Thermo Scientific), SureCycler 8800 Thermal Cyclers (Agilent Technologies), S1000 Thermal Cyclers (Bio-Rad), Eppendorf Mastercycler® (Eppendorf) and Veriti® Thermal Cyclers (Life technologies).

In a particular embodiment, the set of amplification cycles is preceded by a single temperature step at a high temperature, preferably between 90 °C and 100° C, more preferably between 92 °C and 96° C, known as "hold step". Similarly, in a particular embodiment, the set of amplification cycles is followed by an additional step, usually between 4 and 10 °C, preferably 4° C, during the necessary time period for final product brief storage.

In a particular embodiment, wherein the PCR is performed by a DNA polymerase that requires heat activation, an optional initialization step can precede the set of amplification cycles, consisting in a step at a temperature of between 90 °C and 98 °C, more preferably between 94 °C and 96 °C, during 1-20 minutes.

The first step of the amplification cycle is the "denaturation step". This step involves heating of the reaction to a temperature of 90-98 °C, more preferably of 94-98 °C during 10-150 seconds, more preferably during 15-60 seconds, even more preferably during 15-30 seconds. During the denaturation step the template DNA is melted by disruption of the hydrogen bonds between complementary bases, yielding single-stranded DNA molecules.

The second step of the amplification cycle is the "annealing step". This step involves lowering reaction temperature to 45-75 °C, more preferably to 50-65 °C, during 1-240 seconds, more preferably during 15-120 seconds, more preferably during 20-40 seconds. During the annealing step the primers anneal to the single-stranded template DNA molecules. As the skilled person knows, the temperature during the annealing step must be optimised in accordance to the melting temperature of the primers used in the PCR. In general, the annealing temperature is 1-5 °C degrees lower than the melting temperature of the primers used. During this step, DNA molecules are annealed as the result of stable DNA-DNA hydrogen bonds formed only when the primer sequence and the target template sequence match in grade enough. Stable DNA-DNA hydrogen bonds are only formed when the primer sequence very closely matches the template sequence.

The third and last step of the amplification cycle is the "extension step", also known as "elongation step". During this step the polymerase binds to the primer-template hybrid and synthesizes DNA. The temperature at which this step occurs is determined by the particular optimal temperature of the DNA polymerase selected for the PCR. Usual optimal temperatures of DNA polymerases are between 70 and 80 °C, usually between 70 and 75 °C, usually 72 °C between 1 and 300 seconds, preferably between 20 and 90 seconds. At this step, the DNA polymerase synthesizes a new DNA strand complementary to the DNA template strand by adding dNTPs that are complementary to the template in 5' to 3' direction, condensing the 5'-phosphate group of the dNTPs with the 3'-hydroxyl group at the end of the nascent (extending) DNA strand. As the skilled person knows, the extension time during the elongation step depends both on the DNA polymerase used and on the length of the DNA fragment to be amplified. As a rule-of-thumb, at its optimum temperature, the DNA polymerase will polymerize a thousand bases per minute. Under optimum conditions, i.e., if there are no limitations due to limiting substrates or reagents, at each extension step, the amount of DNA target is doubled, leading to exponential (geometric) amplification of the specific DNA fragment.

In a particular embodiment, after the end of the total of PCR cycles, and preceding the storage step at 4° C, an optional final elongation step can be performed, usually performed at a temperature of 70-80 °C, more preferably at 70-74 °C, and for 2-20 minutes, preferably during 2-10 minutes, in order to ensure that any remaining single-stranded DNA is fully extended.

Although, in some particular cases, detectable amplification can be achieved by the method of the invention when the annealing temperature is kept constant along all PCR cycles, in a particular preferred embodiment of the method of the present invention, the PCR occurs in two rounds of amplification cycles, wherein the annealing temperature for the first round of amplification cycles (aT1) is lower than the annealing temperature for the second round of amplification cycles (aT2). As previously mentioned, an amplification cycle relates to a complete sequence of denaturation step, annealing step and elongation step. In a particular embodiment, both the temperature of the denaturation step and the temperature of the elongation step in the first round of amplification cycles according to the PCR-based method of the invention are the same as the temperature of denaturation step and the temperature of elongation step in the second round of amplification cycles, whereas the temperature of the annealing step, also referred to as annealing temperature or binding temperature, in the first round of amplification cycles (aT1) is different from the temperature of the annealing step in the second round of amplification cycles (aT2). In particular, the annealing temperature in the first round of amplification cycles (aT1) is lower that the annealing temperature in the second round of amplification cycles (aT2) [i.e., aT1 < aT2].

The number of amplification cycles in each round (first and second) of amplification cycles can be the same or different and can vary within a broad range. Thus, in a particular embodiment, the first round of amplification cycles comprises between 1 and 30 amplification cycles, more particularly, between 5 and 20 amplification cycles, still more particularly between 10 and 15 amplification cycles; similarly, in a particular embodiment, the second round of amplification cycles comprises between 5 and 50 amplification cycles, more particularly between 10 and 30 amplification cycles, still more particularly between 15 and 25 amplification cycles.

During the annealing step of the first round of amplification cycles, annealing occurs between a target DNA to be amplified and the corresponding complementary region of the primer that targets the DNA sequence to be amplified. During the annealing step of the first round of amplification cycles, the primer tail does not anneal to any target sequence. As a result of the first round of amplification cycles, a number of DNA molecules that include the target DNA and the primer tail are obtained. Thus, the annealing temperature in the first round of amplification cycles is determined by the complementary region of the primer that targets the DNA sequence to be amplified.

During the annealing step of the second round of amplification cycles, annealing occurs between amplified target DNA molecules that have incorporated the primer tail sequence as result from the first round of amplification cycles and both the corresponding complementary region of the primer that targets the DNA sequence to be amplified and the primer tail of the primers. Thus, the annealing temperature in the second round of amplification cycles is determined by the complete primer, both the primer complementary region and the primer tail.

Since only part of the PCR primer hybridizes to the template during the first round of amplification cycles and the complete primer hybridizes to the template during the second round of amplification cycles, i.e. a shorter sequence of the primer hybridizes to the template during the first round of amplification cycles compared to the sequence of the primer that hybridizes to the template during the second round of amplification cycles, the annealing temperature during the first round of amplification cycles is lower than the annealing temperature during the second round of amplification cycles.

As previously mentioned, the annealing temperature depends on the temperature of melting of the primers annealing to the template DNA. Usually, the annealing temperature is 1-5 °C degrees lower than the melting temperature of the primers used. Thus, the skilled person may determine the annealing temperature for the first and second round of amplification cycles based on the sequences of the primers, considering that during the first round of amplification cycles the primer anneals to target DNA along his corresponding complementary region and that during the second round of amplification cycles the primer anneals to DNA along all the sequence of the primer, both the target DNA complementary region and the tail region. Consequently, an increase in the specificity of the PCR-based method of the invention is achieved.

As mentioned above, in a particular embodiment of the PCR-based method of the invention, the annealing temperature is kept constant along all PCR cycles. According to this embodiment, the annealing temperature for all the amplification cycles is comprised between about 45°C and about 75°C, preferably between about 50°C and about 65°C, for example between about 55°C and about 60°C.

In a preferred particular embodiment of the PCR-based method of the invention, the PCR occurs in two rounds of amplification cycles, wherein the annealing temperature for the first round of amplification cycles (aT1) is lower than the annealing temperature for the second round of amplification cycles (aT2). The difference between the annealing temperature for the second round of amplification cycles (aT2) and the annealing temperature for the first round of amplification cycles (aT1) can vary within a broad range, usually between about 1°C and 25°C, preferably between about 5°C and 20°C, more preferably between about 5°C and 15°C. According to this embodiment, the annealing temperature for the first round of amplification cycles (aT1) is comprised between about 45°C and about 75°C, preferably between about 50°C and about 65°C, for example between about 55°C and about 60°C, and the annealing temperature for the second round of amplification cycles (aT2) is comprised between about 45°C and about 75°C, preferably between about 50°C and about 72°C, for example between about 60°C and about 70°C, with the proviso that the annealing temperature for the first round of amplification cycles (aT1) is lower than the annealing temperature for the second round of amplification cycles (aT2) [i.e., aT1 < aT2] as discussed above. (

### PCR products analysis

Following target DNA amplification by PCR according to the invention, the PCR-based method of the invention comprises detection and analysis of said amplified target DNA. According to the method of the invention, if the amplification product size and/or quantity of the nucleic acid target region to be analyzed and amplified is substantially modified when compared to a reference value, then it is indicative of the presence of a gene rearrangement in the nucleic acid region under analysis. In particular, if the size of the amplification product of the target to be analyzed and amplified is increased when compared to a size reference value, then it is indicative of a rearrangement involving an insertion, and wherein if the size of the amplification product of the target to be analyzed and amplified is decreased when compared to a size reference value, then it is indicative of a rearrangement involving a deletion. In particular, if the quantity of the amplification product of the target to be analyzed and amplified is increased when compared to a quantity reference value, then it is indicative of a rearrangement involving an insertion affecting the amplified region, and wherein if the quantity of the amplification product of the target to be analyzed and amplified is decreased when compared to a quantity reference value, then it is indicative of a rearrangement involving a deletion affecting the region amplified. As previously described, in a preferred embodiment, analysis of the quantity of the amplification products involves normalization in respect to the quantity of an amplification product obtained from a DNA region different to the region under rearrangement analysis and comparison of these normalized values with the normalized values obtained in control samples.

In a particular embodiment, the method of the invention involves PCR amplification products resolution and quantification after the PCR has been performed. Resolution of PCR products can be performed by methods known in the art based on the size differences of the PCR amplification products. Thus, in a particular embodiment, PCR products are separated by size and, optionally, their size is determined. PCR products size determination is particularly useful for identification of the different PCR amplification products if a multiplex PCR has been performed. In a particular embodiment, PCR products are quantified. In a particular preferred embodiment, PCR amplification products are separated by size, their size is determined and amplification products are quantified.

Multiple amplification products are simultaneously produced in a multiplex PCR reaction that is then available for simultaneous detection and quantification. Thus, multiple detection signals are inherently produced or emitted that are separately and uniquely detected in one or more detection systems. It is appreciated that multiple detection signals are optionally produced in parallel. Preferably, a single biological sample is subjected to analysis for the simultaneous or sequential detection of genetic sequences. It is appreciated that two or more independent or overlapping sequences are simultaneously or sequentially measured in the process.

Methods for detecting and analyzing PCR amplification products are known in the art and include, without being limited to, gel electrophoresis, Southern blot, mass spectrometry, liquid chromatography, fluorescence, luminescence, gel mobility shift assay, fluorescence resonance energy transfer, nucleotide sequencing, enzyme-linked immunoadsorbent assay, affinity chromatography, chromatography, immunoenzymatic methods, streptavidin-conjugated enzymes, DNA branch migration, enzyme digestion, colorimetric methods, capillary electrophoreses and other kind of electrophoresis, or combinations thereof.

Preferred methods for PCR products separation according to the invention include electrophoresis. Electrophoresis can be performed in gels such as agarose gels or in automated systems such as E-Gel® Agarose Gel Electrophoresis system (Life Technologies). The size of PCR electrophoresed products can be determined by means of molecular weight markers. The selection of the molecular weight marker depends on the expected sizes of the amplification products. Molecular weight markers useful for the invention include, without being limited to, 100bp, 200bp or 1 kb DNA Step Ladders (Promega), 0,05-1,5 kbp, 0,2kpp-15 kbp or 0,1-30 kbp molecular weight markers (Roche Applied Bioscience), etc. Cuantification of PCR electrophoresed products can be performed by quantification ladders. The selection of a particular mass ladder depends on the expected sizes and quantities of the amplification products and include, without being limited to, the HyperLadder I (Bioline, for products ranging from 200 bp to 10 kB in size and between 15 and 100 ng/band in quantitiy), Quanti-Marker (ISC BioExpress, for products ranging from 250 bp to 10 kb in size and between 30 and 100 ng/band in quantity), MassRuler DNA Ladder (Fermentas, for products ranging from 80 bp to 10 kb in size and between 8 and 100 ng/band in quantity) and 2-Log DNA Ladder (New England Biolabs, for products ranging from 200 bp to 10 kb in size and between 15 and 100 ng/band in quantity).

Agarose gel electrophoresis is usually employed for size separation of the PCR products, wherein the size of PCR products is determined by comparison with a DNA ladder (a molecular weight marker), which contains DNA fragments of known size, run on the gel alongside the PCR products. PCR amplification products analysis generates a size estimate relative to the size of the DNA fragments with known lengths of the DNA ladder. Agarose gels may range from 0,5 to 4% agarose, wherein the agarose percentage is determined according to the size of the fragments to be separated (lower agarose percentage for higher PCR products). The agarose gel electrophoresis can be run in buffers such as TBE (89 mM Tris-borate, 2 mM EDTA) or TAE (40 mM Tris-acetate, 2 mM EDTA, pH approx 8.5). In a particular embodiment, the resolved DNA bands are detected by staining the gels with either ethidium bromide (for example, 0.5 µg/mL), followed by destaining with water or SYBR® Green (Molecular Probes Inc., Eugene, OR) and finally photographed under UV illumination.

Other analytical methods to analyze PCR products comprise ethidium bromide-stained 8-10% polyacrylamide gels run in TBE buffer, Southern gels or dot/blots, subcloning and direct sequencing, HPLC analysis, and 96-well microplates. The reverse dot-blot method combines PCR amplification with nonradioactive detection.

PCR products analysis, when said products are labeled with fluorescent dyes, can also be performed by known DNA sequencers as capillary systems from LifeTechnologies or Beckman Coulter.

In a particular preferred embodiment of the PCR-based method of the invention, detection and quantification of PCR amplification products is performed by the Qiaxcel System (Qiagen), which does not involve covalent labeling of primers or PCR products with fluorescent dyes. The QIAxcel system comprises a multicapillary electrophoresis system that can process up to 12 samples per run and can load automatically up to 96 samples. The system consists of the QIAxcel analyzer, a multiplexed fluorescence detection design including an array of light-emitting diodes and micro-optical collectors, QIAxcel Gel Cartridges, BioCalculator Software, and a computer. This system provides fully automated size separation and quantification of DNA fragments in up to 96 samples per run. The QIAxcel technology is based on capillary electrophoresis using gel cartridges. Additional systems according to the invention for fragment analysis and quantification include the Agilent 2100 Bioanalyzer, that comprises a microfluidics-based platform for sizing, quantification and quality control of DNA, RNA, proteins and cells. Results are delivered within 30-40 minutes in automated allowing fragment size detection and fragment quantification automatically.

An alternative procedure for PCR product analysis is the new generation sequencing (NGS), which allows the counting of sequences corresponding to each PCR product, followed by an analysis as described in the present method.

According to the PCR-based method of the present invention, if the amplification product of the target to be analyzed and amplified is substantially modified when compared to a reference value, then it is indicative of the presence of a gene rearrangement in the nucleic acid region under analysis.

Thus, in a particular embodiment, after resolution and quantification of a particular amplification product to be analyzed and amplified, the method of the invention further comprises comparing said quantification value with a reference value, wherein a modification in the quantity of said particular product to be analyzed and amplified with respect to the quantity reference value is indicative of a gene rearrangement.

The reference value can be determined by techniques well known in the state of the art, for example, by determining the quantity level of a PCR amplification product from a particular region comprising the gene rearrangement under study in a sample from a subject who has been previously determined as not carrying the particular gene rearrangement under study.

Quantity levels of PCR amplification products are normalized to a quantity value of a PCR amplification product derived from a genomic region different from the genomic region wherein a particular gene rearrangement is to be determined by the method of the invention. In a particular embodiment, genomic regions different from the genomic region wherein a particular gene rearrangement is to be determined by the method of the invention are those regions comprising genes known in the art as not having rearrangements in normal tissues, causing lethal, premature and/or detectable diseases including, but not limited to, regions of p53, CCNA1, CCND1 and CHRNA1 genes, as well as those genes in different regions when referring to cancer tissues.

According to the invention, PCR amplification products are resolved and analyzed. In a particular embodiment, PCR products are resolved and their size is determined. In a particular preferred embodiment of the invention, PCR products are resolved, their size is determined and their quantity is determined. Thus, in a preferred embodiment, the PCR amplification product of interest to be analyzed and amplified is quantified in a sample from a subject. Once this quantity level is established it can be compared to the reference value, and thus be assigned a level of "decreased" (low), "increased" (high) or "lack of change" quantity level.

An increase in the amplification product to be analyzed and amplified, in particular an increase in its quantity, above the reference value of at least 1.3-fold, 1.4-fold, 1.5-fold or 2-fold, or even more compared with the reference value is considered as "increased" quantity level. On the other hand, a decrease in the amplification product to be analyzed and amplified, in particular a decrease in its quantity, below the reference value of at least 0.7-fold, 0.6-fold, 0.5-fold, 0.02-fold, or even less compared with reference value is considered as "decreased" quantity level. A "lack of change" in the quantity of a particular amplification product to be analyzed and amplified with respect to a reference value refers to quantification levels which are substantially unaltered with respect to the reference value. For instance, a lack of change in the quantity level of a particular amplification product to be analyzed and amplified in a sample under study is considered when the levels differ by no more than 30%, no more than 10%, no more than 5% or no more than 1% or no more than the percentage value that is the same as the error associated to the experimental method used in the determination.

An increased or decreased in the quantity of a particular amplification product to be analyzed and amplified is considered a modification or an alteration in the quantity of said amplification product.

Methods for PCR amplification products resolution and analysis by size and/or quantity have been previously described. In a particular embodiment, PCR products resolution and analysis is represented by a chromatogram, wherein the position of a peak along the x axis is determined by the size of the particular corresponding PCR amplification product and the height and/or the area of a peak in said chromatogram is proportional to the concentration of the particular corresponding PCR amplification product. In an embodiment of the invention, the height of the peak corresponding to a PCR amplification product under analysis is determined and compared to the height of the peak corresponding to the amplification product from a sample known not to have the rearrangement under analysis (reference value), wherein if the height of a PCR amplification product peak is increased when compared to the reference value, it is indicative of a rearrangement involving an insertion, and wherein if the height of a PCR amplification product peak is decreased when compared to the height of the peak corresponding to the amplification product from a sample known not to have the rearrangement under analysis (reference value), it is indicative of a rearrangement involving a deletion. In a preferred embodiment, the area of the peaks corresponding to the PCR amplification products is determined and compared to the area of the peak corresponding to the amplification product from a sample known not to have the rearrangement under analysis (reference value), wherein if the area of a PCR amplification product peak is increased when compared to the reference value, it is indicative of a rearrangement involving an insertion, and wherein if the area of a PCR amplification product peak is decreased when compared to the area of the peak corresponding to the amplification product from a sample known not to have the rearrangement under analysis (reference value), it is indicative of a rearrangement involving a deletion.

In an embodiment of the invention, the modification in a particular amplification product to be analyzed and amplified is an increase or a decrease in the amplification product of the region under study. tha gene selected from the group comprising the genes ABCA1, ABCB11, ABCG5, ABCG8, ANK2, APOA1, APOB, APOC2, APOE, ARH, ATP8B1, BRCA1, BRCA2, CBS, CETP, CFTR, CLCNKB, CLDN16, CLDN19, COL1A1, COL1A2, COL3A1, DAX1, DMGDH, FBN1, FLCN, FMO1, FMO3, GCK, HNF1A, HNF1B, HNF4A, KCNE1, KCNE2, KCNH2, KCNJ2, KCNQ1, LCAT, LDLR, LPL, MEFV, menin, MLH1, MSH2, MTHFR, MTP, NF1, NF2, PCSK9, PKP2, PRKAR1A, PRSS1, RET, SCN5A, SDHB, SDHC, SDHD, SERPINA1, SLC12A3, SLC22A1, SLC34A2, SPINK1, TGFBR1 and TGFBR2.

In a particular embodiment of the invention, the modification in a particular amplification product to be analyzed and amplified is an increase or a decrease in the amplification product of a region of the LDLR gene with respect to a reference value. In another particular embodiment, the modification in a particular amplification product to be analyzed and amplified is an increase or a decrease in the amplification product of a region of the BRCA1 gene with respect to a reference value.

As the skilled person can understand, the method according to the invention can be useful in the diagnosis of diseases and disorders caused by large rearrangements in one or more genes. Examples of diseases and disorders involving large rearrangements that could be subject of diagnosis by the method of the invention include, but are not limited to:
- chromosome rearrangement including, but not limited to, related diseases and disorders to chromosome rearrangements such as, idiopathic acquired sideroblastic anemia, pancytopenia, hereditary persistence of fetal hemoglobin, attention deficit hyperactivity disorder, juvenile nephronophthisis, autosomal dominant polycystic kidney disease, cancer such as retinoblastoma, sarcoma, fibroadenoma, Wilms tumou, malignant mixed mesodermal tumor, gonadoblastoma, pleomorphic adenoma, hematologic cancer, germ cell tumors, aggressive carcinoma, neuroblastoma, vestibular schwannoma, malignant peripheral nerve sheath tumor, primitive neuroectodermal tumor, small cell lung carcinoma, melanoma, Ewing tumor, glial tumors, meningioma, paraspinal neuroblastoma, glioblastoma multiforme, low-grade astrocytoma, supratentorial ependymoma, glioblastoma, Lynch syndrome, prostate carcinoma, paratesticular rhabdomyosarcoma metastatic to lungs and bone, ovarian cancer, endometrial stromal sarcoma, bilateral retinoblastom, clear cell sarcoma of kidney, uterine leiomyoma, autosomal dominant polycystic kidney disease, head and neck cancer, papillary thyroid carcinomas, breast cancer, lymphocytic leukemia, funicular lymphoma, leukemia, Hodgkin disease, myeloid leukemia, large cell lymphoma, Burkitt lymphoma, diffuse large-cell lymphoma, multiple myeloma, hereditary breast cancer and mesenchymal hamartoma of liver, Severe Myoclonic Epilepsy of Infancy, lymphoproliferative disorders such as aggressive NK cell leukemia, B-cell chronic lymphocytic leukemia, T cell leukemia, acute lymphoblastic leukaemia, B-cell lymphoma, lymphoblastic lymphoma, acute lymphoblastic leukaemia, T cell neoplasia, posttransplant B-cell lymphoma and T-cell cutaneous lymphoma, mental retardation such as Lowe syndrome, lubs X-linked mental retardation syndrome, severe mental retardation, *cri du chat* syndrome, tuberous sclerosis (tsc), Down syndrome, mild mental retardation range, Klinefelter syndrome, coffin-siris syndrome, Williams-Beuren syndrome, Angelman syndrome, mesomelic dysplasia such as osteogenesis imperfecta and cleidocranial dysplasia, muscular dystrophy such as myotonic dystrophy, Duchenne muscular dystrophy and Emery-Dreifuss muscular distrophy, psychotic disorders such as Rett syndrome, autism and schizophrenia, psychomotor retardation such as Gilles de la Tourette syndrome and apraxia of speech, reproductive failures such as ovarian cancer, endometrial stromal sarcoma, bilateral retinoblastoma, malignant mixed mesodermal tumor, uterine leiomyoma, breast cancer, prostate carcinoma, paratesticular rhabdomyosarcoma metastatic to lungs and bone, Ullrich-Turner syndrome, Klinefelter syndrome, primary hypogonadism, premature ovarian failure, amenorrhoea, aggressive angiomyxoma of vulva, hereditary persistence of fetal hemoglobin, hypomagnesemia with secondary hypocalcemia, Smith-Magenis syndrome and moyamoya disease, azoospermia and infertility, supravalvular aortic stenosis such as Williams-Beuren syndrome, Von Recklinghausen neurofibromatosis such as neurofibromatosis type I and others such as those disclosed in the database dbCRID at http://dbcrid.biolead.org/phenotype.php (Kong F et al. 2010 Nucleic Acids Res 39: D895-D900).
- cytogenetic abnormalities related diseases and disorders including, but not limited to, monosomies such as Turner syndrome, *cri du chat* syndrome and 1p36 deletion syndrome, trisomies such as Down syndrome (trisomy 21), Edwards syndrome (trisomy 18), Patau syndrome (trisomy 13), trisomy 9, Warkany syndrome 2, trisomy 22, triple X syndrome (XXX), Klinefelter's syndrome (XXY) and XYY;
- CNV polymorphisms related diseases and disorders including, but not limited to, attention deficit hyperactivity disorder (ADHD), autism, schizophrenia.

In a particular embodiment of the invention, the modification in a particular amplification product to be analyzed and amplified is an increase or a decrease in the amplification product of a region of the LDLR gene with respect to a reference value. Thus, the method of the invention can be useful in the diagnosis of diseases and/or disorders involving gene rearrangements in the LDLR gene including, without being limited to, familial hypercholesterolemia included in the autosomal dominant hypercholesterolemias or ADHs, an autosomal dominant disease characterized by elevated concentrations of LDL in blood plasma.

In another particular embodiment, the modification in a particular amplification product to be analyzed and amplified is an increase or a decrease in the amplification product of a region of the BRCA1 gene with respect to a reference value. Thus, the method of the invention can be useful in the diagnosis of diseases and/or disorders involving gene rearrangements in the BRCA1 gene such as including, without being limited to, hereditary and sporadic breast cancer and ovarian cancer.

Additional applications of the method of the invention include prenatal and/or postnatal studies for chromosomal alterations, such as trysomy of chromosome 21 (Down syndrome), trisomy of chromosome 18 (Edward syndrome), trisomy of chromosome 13 (Patau syndrome), Klinefelter syndrome (XXY) or Turner syndrome (XO). Additional applications of the method of the invention also relate to the detection of minor insertions and/or deletions included in an amplicon, pharmacogenetics and methylation analysis.

### Kit of the invention

In an additional aspect, the invention is related to a kit that comprises a reagent adequate for detection of rearrangements in a nucleic acid sequence, wherein said reagent comprises PCR primers with two regions:
- a first region, which is not complementary to the nucleic acid target sequence to be analyzed for rearrangements, located at the 5' end of the PCR primer, and
- a second region, which is complementary to the nucleic acid target sequence to be analyzed for rearrangements, located at the 3' end of the PCR primer.

The term "reagent adequate for detection of rearrangements in a nucleic acid sequence" means a compound or set of compounds that allows determining the presence of rearrangements in a nucleic acid sequence by means of amplification of a target region susceptible of comprising said particular rearrangement. Thus, reagents include PCR primers capable of specifically hybridizing with the target region susceptible of comprising a particular rearrangement under analysis.

PCR primers according to the kit of the invention comprise two regions, a first region, which is not complementary to the nucleic acid target sequence to be analyzed for rearrangements, located at the 5' end of the PCR primer, and a second region, which is complementary to the nucleic acid target sequence to be analyzed for rearrangements, located at the 3' end of the PCR primer. PCR primers according to the kit of the invention have been previously described in detail in the context of the method of the invention.

In a particular embodiment, PCR primers according to the kit of the invention are used in a multiplex PCR. Thus, in a particular embodiment, the kit comprises reagents comprising at least two primer pairs, a first primer pair for amplification of the region wherein the presence of a rearrangement is to be analyzed and a second primer pair for amplification of a control region. In a preferred embodiment of the invention, the kit comprises reagents comprising at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 primer pairs, at least 25, at least 30, at least 35, at least 40, at least 45 or at least 50 primer pairs, wherein at least one primer pair is for amplification of a control region.

In a particular embodiment, the kit of the invention comprises reagents for detection of rearrangements, preferably of rearrengements involved in diseases and disorders. In a particular embodiment, the kit of the invention comprises reagents for detection of rearrangements in at least one of the genes selected from the group comprising the genes ABCA1, ABCB11, ABCG5, ABCG8, ANK2, APOA1, APOB, APOC2, APOE, ARH, ATP8B1, BRCA1, BRCA2, CBS, CETP, CFTR, CLCNKB, CLDN16, CLDN19, COL1A1, COL1A2, COL3A1, DAX1, DMGDH, FBN1, FLCN, FMO1, FMO3, GCK, HNF1A, HNF1B, HNF4A, KCNE1, KCNE2, KCNH2, KCNJ2, KCNQ1, LCAT, LDLR, LPL, MEFV, menin, MLH1, MSH2, MTHFR, MTP, NF1, NF2, PCSK9, PKP2, PRKAR1A, PRSS1, RET, SCN5A, SDHB, SDHC, SDHD, SERPINA1, SLC12A3, SLC22A1, SLC34A2, SPINK1, TGFBR1 and TGFBR2, more particularly, at least one primer pair for detection of rearrangements in at least one of the mentioned genes. In a particular embodiment, the kit of the invention comprises reagents for detection of rearrangements in at least one of the genes selected from the group comprising the genes BRCA1, COL1A1, COL1A2, COL3A1, CTFR, LDLR, menin, NF1 and NF2, more particularly, at least one primer pair for detection of rearrangements in at least one of the mentioned genes. In a particular embodiment, the kit of the invention comprises reagents for detection of rearrangements in the LDLR gene, more particularly, at least one PCR primer pair for detection of rearrangements in the LDLR gene. In another particular embodiment, the kit of the invention comprises reagents for detection of rearrangements in the BRCA1 gene, more particularly, at least one PCR primer pairs for detection of rearrangements in the BRCA1 gene.

Accordingly, the kit of the invention can be used in the diagnosis of diseases and disorders involving gene rearrangement. Examples of diseases and disorders involving large rearrangements have been previously mentioned in the context of the method of the invention. In a particular embodiment, the diseases and disorders involving gene arrangement are selected from the group comprising familial hypercholesterolemia, breast cancer and ovarian cancer.

The invention is described in detail below by means of the following examples which are to be construed as merely illustrative and not limitative of the scope of the invention. Examples 1 and 2 show that rearrangements in the *LDLR* gene and the *BRCA1* gene have been successfully detected by the method of the invention.

### EXAMPLES

### Materials and methods

### PCR general method

The method of the invention is based on a PCR reaction for amplification of the regions of interest from test samples. PCR products are loaded into a system for DNA fragments analysis and quantification. Once the quantification is performed, a series of simple calculations are made in order to determine the presence or absence of large rearrangements. An scheme of the general process of the working procedure according to the method of the invention is shown in Fig. 1.

### PCR for LDLR gene analysis

The QIAGEN Multiplex PCR kit was used for performing the PCR. The following conditions were used:

| | |
|---|---|
| PCR reaction mix: | 7.5 µL |
| Water: | 4.5 µL |
| Oligonucleotide mix (5 µM): | 1 µL |
| Template DNA (25 ng/µL): | 1.5 µL |
| TOTAL VOLUME (per well): | 14.5 µl |

The following thermocycler conditions were used during the PCR:

| | | |
|---|---|---|
| 95 | 15 min | x 1 cycle |
| 95 | 20 sec | |
| 60 | 20 sec | x 10 cycles |
| 72 | 45 sec | |
| 95 | 20 sec | |
| 65 | 20 sec | x 20 cycles |
| 72 | 45 sec | |
| 72 | 3 min | x 1 cycle |
| 15 | 00 | |

PCR products were loaded onto the QIAexcel System (Qiagen).

### PCR for BRCA1 gene analysis

The PCR mixture was prepared as previously described for LDLR gene analysis. Thermocycler conditions were also the same as those used for LDLR receptor. PCR products were loaded onto a QIAexcel System (Qiagen) after the PCR.

### PCR primers

PCR primers are designed so as to comprise a "tail" which does not hybridize with the template DNA. A common and specific tail is added to 5'end of forward primers, and a common and specific tail is added to the 5' end of reverse primers, which is different from the tail of the forward primers. The presence of these tails or modifications of PCR primers allows a PCR that is more specific than standard PCR. Thus, in a first round of amplification cycles, the annealing temperature of the complementary sequences of the primers to the DNA is used; in a second set of cycles, the annealing temperature corresponds to the temperature calculated for the entire sequence of the primers, tail included. A scheme of this amplification process is shown in Figure 2.

The sequence of the primer tails may vary depending on the particular design to be performed (both in size and sequence). The main function of these primer tails is to allow specific primer binding in the first round of amplification cycles, and to homogenize the amplification in the second round of amplification cycles due to the increase of the melting temperature (Tm) of the primers and the common regions of the primers.

PCR primer pairs used for LDLR gene analysis are shown in Table 1. PCR primer pairs for BRCA1 gene analysis are shown in Table 2.

**Table 1. PCR primer pairs in LDLR gene analysis**

| Primer name | Sequence (5'-3') | SEQ ID NO |
|---|---|---|
| QxRLDL-E15-5unv | *AGGTCAGGATCAACGCTCAAG*AGGCACGTGGCACTCAGAAG | 1 |
| QxRLDL-E15-3unv | *CATCTTGCATGATCCAACCTTC*GACCCGTCTCTGGGTGAAGAG | 2 |
| QxRLDL-E4-5unv | *AGGTCAGGATCAACGCTCAAG*CAGCTTCCAGTGCAACAGCTC | 3 |
| QxRLDL-E4-3unv | *CATCTTGCATGATCCAACCTTC*CAGTTTTCCTCGTCAGATTTGTCC | 4 |
| QxRLDL-E5-5unv | *AGGTCAGGATCAACGCTCAAG*TGTCCTGTTTTCCAGCTGTGG | 5 |
| QxRLDL-E5-3unv | *CATCTTGCATGATCCAACCTTC*AAATCATTTGCAAGCAGCAAGG | 6 |
| Qx-p53_E-5unv | *AGGTCAGGATCAACGCTCAAG*AAGCAGTCACAGCACATGACG | 7 |
| Qx-p53_E-3unv | *CATCTTGCATGATCCAACCTTC*AATTTCCTTCCACTCGGATAAGATG | 8 |
| QxRLDL-E7-5unv | *AGGTCAGGATCAACGCTCAA*GGTTCCCACGTCTGCAATGAC | 9 |
| QxRLDL-E7-3unv | *CATCTTGCATGATCCAACCTTC*ACTTAACAGATGGGGAAACTGAGG | 10 |
| QxRLDL-E13-5unv | AGGTCAGGATCAACGCTCAAGGTGTTTAACGGGATTTGTCATCTTC | 11 |
| QxRLDL-E13-3unv | *CATCTTGCATGATCCAACCTTC*TATCCTCTGGGGACAGTAGGTTTTC | 12 |
| QxRLDL-E18-5unv | *AGGTCAGGATCAACGCTCAAG*GATAGTTTCCGCTGTTTACCATTTG | 13 |
| QxRLDL-E18-5unv | *CATCTTGCATGATCCAACCTTC*AGGTCTCAGGAAGGGTTCTGG | 14 |
| QxRLDL-PrC-5uv | *AGGTCAGGATCAACGCTCAAG*AGTTTGCAAAATCCCAGAGAAATC | 15 |
| QxRLDL-PrC-3uv | *CATCTTGCATGATCCAACCTTC*AAAGGCACATGTGAGAAACTTGC | 16 |
| QxRLDL-PrA-5uv | AGGTCAGGATCAACGCTCAAGCATTCGTGAAATCTAACTAGGAGTAG TCTG | 17 |
| QxRLDL-PrA-3uv | CATCTTGCATGATCCAACCTTCTGAATTGTATCTGTGTGAAACACAT TG | 18 |
| QxRLDL-E3-5n | *AGGTCAGGATCAACGCTCAAG*CCTCAGTGGGTCTTTCCTTTG | 19 |
| QxRLDL-E3-3n | *CATCTTGCATGATCCAACCTTC*GACCCCGTAGAGACAAAGTCAG | 20 |
| QxRLDL-E2-5 | *AGGTCAGGATCAACGCTCAAG*AAATAGACACAGGAAACGTGGTCAG | 21 |
| QxRLDL-E2-3 | *CATCTTGCATGATCCAACCTTC*AAATAAATGCATATCATGCCCAAAG | 22 |
| qxRLDL-E9-5 | *AGGTCAGGATCAACGCTCAAG*GTCAGGAAGATGACGCTGGAC | 23 |
| qxRLDL-E9-3 | *CATCTTGCATGATCCAACCTTC*AGGAGGAGAGAAGGGCATCAG | 24 |
| QxRLDL-E11-5 | *AGGTCAGGATCAACGCTCAAG*TTTCTTCCAGAATTCGTTGCAC | 25 |
| QxRLDL-E11-3 | *CATCTTGCATGATCCAACCTTC*ATTGGGCCACTGAATGTTTTC | 26 |
| Qx-p53_D-5 | *AGGTCAGGATCAACGCTCAAG*GCTCAAGACTGGCGCTAAAAG | 27 |
| Qx-p53_D-3 | *CATCTTGCATGATCCAACCTTC*AGAGGACTCATCAAGTTCAGTCAGG | 28 |
| QxRLDL-E8-5 | *AGGTCAGGATCAACGCTCAAG*CTACAAGTGCCAGTGTGAGGAAG | 29 |
| QxRLDL-E8-3 | *CATCTTGCATGATCCAACCTTC*TATGAGTCTGTGCAAAGTTCAGAGG | 30 |
| QxRLDL-E17-5 | *AGGTCAGGATCAACGCTCAAG*GAGCTGGGTCTCTGGTCTCG | 31 |
| QxRLDL-E17-3 | *CATCTTGCATGATCCAACCTTC*GTCAAAGTTGATGCTGTTGATGTTC | 32 |
| QxRLDL-I1B-5uv | AGGTCAGGATCAACGCTCAAGAATAATTTGTTATTGCTTTTATTGCC TTAG | 33 |
| QxRLDL-I1B-3uv | CATCTTGCATGATCCAACCTTCAATACCTTTATATGAAAGATTCTGG TTCTG | 34 |
| QxRLDL-E1n-5uv | *AGGTCAGGATCAACGCTCAAG*GAGGTGAAGACATTTGAAAATCACC | 35 |
| QxRLDL-E1n-3uv | CATCTTGCATGATCCAACCTTCGTCATTTACAGCATTTCAATGTGAG G | 36 |

| | | |
|---|---|---|
| E: exon; Pr: promoter. Underlined primer sequence corresponds to the region complementary to LDLR gene. Primer sequence *in italics* corresponds to the tail region of the primer. | | |

**Table 2. PCR primer pairs in BRCA1 gene analysis**

| Primer name | Sequence | SEQ ID NO |
|---|---|---|
| P53-215-5 | *AGGTCAGGATCAACGCTCAAG*CTTCTTGCATTCTGGGACAGC | 37 |
| P53-215-3 | *CATCTTGCATGATCCAACCTTC*ATTCAACTTTGGGACAGGAGTCAG | 38 |
| P53-450-5 | *AGGTCAGGATCAACGCTCAAG*AGGTAGGACCTGATTTCCTTACTGC | 39 |
| P53-450-3 | *CATCTTGCATGATCCAACCTTC*CAGGTAAAACAGTCAAGAAGAAAACG | 40 |
| PR5 | *AGGTCAGGATCAACGCTCAAG*AGAAGATTGGCTCTTACCACTTGTC | 41 |
| PR-3 | *CATCTTGCATGATCCAACCTTC*CGTATTTTGAAAGCAGAAACTAGGC | 42 |
| E2-5 | *AGGTCAGGATCAACGCTCAAG*CGCGTTGAAGAAGTACAAAATGTC | 43 |
| E2-3 | *CATCTTGCATGATCCAACCTTC*TCTTCCCTAGTATGTAAGGTCAATTCTG | 44 |
| E3-5 | *AGGTCAGGATCAACGCTCAAG*CAGTTCCTGACACAGCAGACATTT | 45 |
| E3-3 | *CATCTTGCATGATCCAACCTTC*TCTGAGAAAGAATGAAATGGAGTTG | 46 |
| E4-5 | *AGGTCAGGATCAACGCTCAAG*TGCATGTAAGTTCAGTTTTCATAGATCA | 47 |
| E4-3 | *CATCTTGCATGATCCAACCTTC*CTGTGAAGGCCCTTTCTTCTG | 48 |
| E5-5 | *AGGTCAGGATCAACGCTCAAG*GAGGTTTTCTACTGTTGCTGCATCT | 49 |
| E5-3 | *CATCTTGCATGATCCAACCTTC*TAAGGTGTGAGACCAGTGGGAGTA | 50 |
| E7-5 | *AGGTCAGGATCAACGCTCAAG*CAATTTATTTTGTCCATGGTGTCA | 51 |
| E7-3 | *CATCTTGCATGATCCAACCTTC*ACCACAAATACAAATTATGACCAAGAT | 52 |
| E8-5 | *AGGTCAGGATCAACGCTCAAG*AAAGAGAACCTTTGTCTATGAAGCTG | 53 |
| E8-3 | *CATCTTGCATGATCCAACCTTC*TACACCAAATCCCAAGTCGTG | 54 |
| E9-5 | *AGGTCAGGATCAACGCTCAAG*CCTATAGTGTGGGAGATCAAGAATTG | 55 |
| E9-3 | *CATCTTGCATGATCCAACCTTC*TCTCTTTTCAGTGCCTGTTAAGTTG | 56 |
| E10-5 | *AGGTCAGGATCAACGCTCAAG*GTTCAGAGGCAACGAAACTGG | 57 |
| E10-3 | *CATCTTGCATGATCCAACCTTC*TCCAGGAAGACTTTGTTTATAGACCTC | 58 |
| E10B-5 | *AGGTCAGGATCAACGCTCAAG*CTAACCAAACGGAGCAGAATGG | 59 |
| E10B-3 | *CATCTTGCATGATCCAACCTTC*TTTGAACACTTAGTAAAAGAACCAGGTG | 60 |
| E11-5 | *AGGTCAGGATCAACGCTCAAG*GGTGTCCTTTGCATTCAGTAGTATG | 61 |
| E11-3 | *CATCTTGCATGATCCAACCTTC*TGGGTTTCGAAGGTTTAGCTTATTC | 62 |
| E12-5 | *AGGTCAGGATCAACGCTCAAG*TTCATTTAATGGAAAGCTTCTCAAAGT | 63 |
| E12-3 | *CATCTTGCATGATCCAACCTTC*GGGAAGGAAAGAATTTTGCTTAAGAT | 64 |
| E13-5 | *AGGTCAGGATCAACGCTCAAG*TGTCTGTTGCATTGCTTGTGTTTA | 65 |
| E13-3 | *CATCTTGCATGATCCAACCTTC*CGCACATTTCTCATGTTGTAGCTT | 66 |
| E14-5 | *AGGTCAGGATCAACGCTCAAG*TTTGCCAGTCATTTCTGATCTCTC | 67 |
| E14-3 | *CATCTTGCATGATCCAACCTTC*CAAAGTGTTTGTTCCAATACAGCAG | 68 |
| E15-5 | *AGGTCAGGATCAACGCTCAAG*GAGGAAGAAATCTAGAAAACAATCACAG | 69 |
| E15-3 | *CATCTTGCATGATCCAACCTTC*TGGGGAATAAAACTATACCTACTTGC | 70 |
| E16-5 | *AGGTCAGGATCAACGCTCAAG*CCAGAAAACACCACATCACTTTAAC | 71 |
| E16-3 | *CATCTTGCATGATCCAACCTTC*TGCAAGGTATTCTGTAAAGGTTCTTG | 72 |
| E18-5 | *AGGTCAGGATCAACGCTCAAG*AGCACGTTCTTCTGCTGTATGTAAC | 73 |
| E18-3 | *CATCTTGCATGATCCAACCTTC*ATTTGTGCATTGTTAAGGAAAGTGG | 74 |
| E19-5 | *AGGTCAGGATCAACGCTCAAG*GAGGTTTCACCATGTTGGTCAG | 75 |
| E19-3 | *CATCTTGCATGATCCAACCTTC*TAAAGTCCCAGCTCTTCCACTTATC | 76 |
| E20-5 | *AGGTCAGGATCAACGCTCAAG*TCCCTCTCTCTTCCTCTCTTCTTC | 77 |
| E20-3 | *CATCTTGCATGATCCAACCTTC*CTGGAACTCTGGGGTTCTCC | 78 |
| E21-5 | *AGGTCAGGATCAACGCTCAAG*CAGTGGCAAATTGACTTAAAATCC | 79 |
| E21-3 | *CATCTTGCATGATCCAACCTTC*GGAGTCTTTTGGCACAGGTATGT | 80 |
| e22-5 | *AGGTCAGGATCAACGCTCAAG*TTTGACACTTTGAATGCTCTTTCC | 81 |
| E22-3 | *CATCTTGCATGATCCAACCTTC*ATTGTCCTCTGTCCAGGCATC | 82 |
| E23-5 | *AGGTCAGGATCAACGCTCAAG*AATCCTAAGAACTCATACAACCAGGAC | 83 |
| E23-3 | *CATCTTGCATGATCCAACCTTC*GTAGAGTGCTACACTGTCCAACACC | 84 |

| | | |
|---|---|---|
| E: exon; Pr: promoter. Underlined primer sequence corresponds to the region complementary to BRCA1 gene. Primer sequence *in italics* corresponds to the tail region of the primer. | | |

As shown in Figure 3, corresponding to LDLR gene analysis, the combination of modified oligonucleotides and cycles at different temperatures according to the PCR-based method of the invention makes the results much more reproducible and the protocol much simpler to optimize, since the results obtained are more homogenous between fragments. However, it is mainly the oligonucleotide modification characteristic of the method according to the invention that allows an easier and more reproducible detection of the presence of large rearrangements, since intensity differences are more marked and less dependent on the sequence of each oligonucleotide and fragment, as it is shown in Figure 4.

### PCR controls

The PCR reaction also includes the amplification of one or several DNA control fragments from a different gene or genes than the one under study. This allows the normalization of the results, by dividing the intensity of each peak by the intensity of the control peak. Consequently, the intensity of each fragment is seen in relation to that of the control fragment. In the analysis of LDLR and BRCA1 genes, different regions of the p53 gene have been used as control fragments.

Another important control that is added to independent reactions is control samples for calculating the normal values for each fragment. Variations from the median of the control samples superior to 25-30% for a fragment in the problem sample indicate the presence of insertions (when it is an increase) or a deletion (when it is a decrease) (see Figure 5). In the analysis of LDLR and BRCA1 genes, samples from three healthy individuals have been used.

The analysis using the Qiaxcel system allows the identification of every one of the amplified fragments by their length and their automatically quantification. The use of a simple spreadsheet allows the normalization of the fragments of interest with that for the controls. Thus, the variation of each fragment in relation to the control is obtained (Figures 5 and 6).

### Comparison with other methods

When compared to other protocols that require several days to obtain an end result and many dedicated hours, the method of the invention allows detection of large rearrangements (as well as other types of genetic modifications) in a few hours and with a minimum of work (three pipettings and two manipulations; PCR and Qiaxcel loading), see Figure 1. Other methods known in the art require much more work and time. For example, MLPA (a commercial method that is simpler than other previous techniques) requires 25-30 pipetting steps and five sample manipulations (denaturalization, hybridization, ligation, PCR and sequencer loading) after which the sequencer analyses the sample, for a total of three days to obtain the final result, including six hours of work.

### Example 1. Detection of mutations in LDLR gene

Large mutations are present in about 14% of patients affected by familial hypercholesterolemia (Simard LR et al. 2004 Clin Genet 65:202-208). Indirect diagnosis of familial hypercholesterolemia has been described in the art by means of STR (short tandem repeats) and polymorphism analysis. Detection of point mutations has been previously performed by SSCPs (single strand conformation polymorphism) and DNA sequencing of altered fragments (nowadays it is normal to sequence all the exons directly). Large mutation or rearrangements have been also described to be detected by Southern blot.

First, in order to analyze the LDLR gene, oligonucleotides that permit amplifying DNA fragments of different sizes have been designed by the inventors. Amplification conditions for normal and modified oligonucleotides have been optimized along with creating the best conditions in order to detect changes in 50% of the DNA template present in the reaction. Second, it has been determined that the results were reproducible, both within as well as between experiments, since the use of internal controls made it possible to be sure that no information was lost in each one of them. Figure 7 shows the results of amplifying 3 different samples in three different reactions each one. The standard deviation is less than 2% in the first experiments with modified oligonucleotides and after the protocol modifications, it is less than 1%.

It has been verified that the system can detect large rearrangements along the entire length of the LDLR gene. This is supported by the analysis of samples with these modifications (see Figures 4, 5 and 6). Comparing the effectiveness of the two types of oligos used in the detection of real mutations, the modified oligos were much better (Figure 4). To assure that the results were independent from the observer; one person selected 36 samples analyzed by Southern Blot for the presence of large rearrangements. Another person who was ignorant of the large rearrangements identified by the inventors and of the samples to be studied, performed the study of the samples and analyzed the results. The results of the latter study coincided completely with those of the former study, detecting large rearrangements in 23 of the samples and excluding them in 13.

The inventors have developed a simple spreadsheet that allows the detection of the presence of large rearrangements for a gene either by studying the variation in the intensity (variations above 25%) or by analyzing the graph created by the spreadsheet (Figure 6).

### Example 2. Detection of mutations in BRCA1 gene

An additional study was designed for a second gene that frequently presents large rearrangements, *BRCA1*. The protocol allows the analysis of the complete gene using a multiplex of 25 fragments (Figure 8), showing detection of all the large rearrangements previously found by using other techniques.

## Claims

1. A PCR-based method for the detection of a rearrangement in a nucleic acid in a sample from a subject, wherein each PCR primer comprises two regions:
- a first region, which is not complementary to the nucleic acid target region to be analyzed and amplified, located at the 5' end of the PCR primer, and
- a second region, which is complementary to the nucleic acid target region to be analyzed and amplified, located at the 3' end of the PCR primer,
wherein the amplification product size and/or quantity of the nucleic acid target region to be analyzed and amplified, in the absence of a rearrangement, is determined and considered as the reference value, and
wherein if the amplification product size and/or quantity of the nucleic acid target region to be analyzed and amplified in said sample is substantially modified when compared to the reference value, then it is indicative of the presence of a gene rearrangement in the nucleic acid in said sample.

2. The method according to claim 1 wherein the PCR occurs in two rounds of amplification cycles, so as the annealing temperature for the first round of amplification cycles (aT1) is lower that the annealing temperature for the second round of amplification cycles (aT2).

3. The method according to any of the preceding claims wherein the size of the amplification product to be analyzed and amplified is determined, wherein if the amplification product size is increased when compared to a size reference value, then it is indicative of a rearrangement involving an insertion, and wherein if the amplification product size is decreased when compared to a size reference value, then it is indicative of a rearrangement involving a deletion.

4. The method according to any of the preceding claim wherein the rearrangement involves a region of 1 bp to 100 kpb.

5. The method according to any of the preceding claims wherein the amplification product to be analyzed and amplified is quantified, wherein if the amplification product quantity is increased when compared to a quantity reference value, then it is indicative of a rearrangement involving an insertion, and wherein if the amplification product quantity is decreased when compared to a quantity reference value, then it is indicative of a rearrangement involving a deletion.

6. The method according to the preceding claim wherein the rearrangement involves a region of 100 bp to 100 kpb.

7. The method according to any of the preceding claims wherein the PCR-based method is a multiplex PCR.

8. The method according to any of the preceding claims wherein rearrangements in the nucleic acid are selected from the group comprising cytogenetic abnormalities, chromosome rearrangements and CNV polymorphisms.

9. The method according to any of the preceding claims wherein chromosome rearrangements are selected from the group comprising deletions, duplications, insertions, inversions, translocations, and transpositions.

10. A method according to any of the preceding claims wherein the nucleic acid target region is a region of a gene selected from the group comprising the genes ABCA1, ABCB11, ABCG5, ABCG8, ANK2, APOA1, APOB, APOC2, APOE, ARH, ATP8B1, BRCA1, BRCA2, CBS, CETP, CFTR, CLCNKB, CLDN16, CLDN19, COL1A1, COL1A2, COL3A1, DAX1, DMGDH, FBN1, FLCN, FMO1, FMO3, GCK, HNF1A, HNF1B, HNF4A, KCNE1, KCNE2, KCNH2, KCNJ2, KCNQ1, LCAT, LDLR, LPL, MEFV, menin, MLH1, MSH2, MTHFR, MTP, NF1, NF2, PCSK9, PKP2, PRKAR1A, PRSS1, RET, SCN5A, SDHB, SDHC, SDHD, SERPINA1, SLC12A3, SLC22A1, SLC34A2, SPINK1, TGFBR1 and TGFBR2.

11. A method according to any of the preceding claims wherein the rearrangement is in heterozygosis in the subject.

12. A kit comprising a reagent adequate for detection of rearrangements in a nucleic acid sequence, wherein said reagent comprises PCR primers with two regions:
- a first region, which is not complementary to the nucleic acid target sequence to be analyzed for rearrangements, located at the 5' end of the PCR primer, and
- a second region, which is complementary to the nucleic acid target sequence to be analyzed for rearrangements, located at the 3' end of the PCR primer.

13. A kit according to the preceding claim wherein the reagent comprises PCR primers for detection of rearrangements in any gene or genetic region selected from the group comprising the genes ABCA1, ABCB11, ABCG5, ABCG8, ANK2, APOA1, APOB, APOC2, APOE, ARH, ATP8B1, BRCA1, BRCA2, CBS, CETP, CFTR, CLCNKB, CLDN16, CLDN19, COL1A1, COL1A2, COL3A1, DAX1, DMGDH, FBN1, FLCN, FMO1, FMO3, GCK, HNF1A, HNF1B, HNF4A, KCNE1, KCNE2, KCNH2, KCNJ2, KCNQ1, LCAT, LDLR, LPL, MEFV, menin, MLH1, MSH2, MTHFR, MTP, NF1, NF2, PCSK9, PKP2, PRKAR1A, PRSS1, RET, SCN5A, SDHB, SDHC, SDHD, SERPINA1, SLC12A3, SLC22A1, SLC34A2, SPINK1, TGFBR1 and TGFBR2.

14. Use of a kit according to any of claims 12 or 13 in the diagnosis of diseases and disorders involving gene rearrangement.

15. Use of a kit according to claim 14 wherein diseases and disorders involving gene rearrangement are selected from the group comprising familial hypercholesterolemia, breast cancer and ovarian cancer.
